# EUROPEAN PATENT APPLICATION

(11) **EP 3 498 266 A1**
(43) Date of publication of application: **19.06.2019**
(21) Application number: 17207771.1
(22) Date of filing: 15.12.2017
(51) Int. Cl.: A61K 9/19, A61K 47/02, A61K 47/26

(54) **FORMULATIONS OF COPANLISIB**

(71) Applicant: Bayer Consumer Care AG, 4002 Basel (CH); Bayer Pharma Aktiengesellschaft, 13353 Berlin (DE)
(72) Inventor: Freundlieb, Julia, 44229 Dortmund (DE)
(74) Representative: BIP Patents

(57) **Abstract**

The present invention relates to :
• a method of preparing a stable, copanlisib-containing aqueous bulk solution suitable for lyophilisation and for therapeutic applications ;
• a stable, copanlisib-containing bulk solution, of increased solubility which is directly suitable for lyophilisation ;
• a method of lyophilizing such a copanlisib-containing aqueous bulk solution ;
• a stable, lyophilized, copanlisib-containing solid, in particular powder or cake, particularly containing a therapeutic dose of copanlisib in one or two containers, particularly one sealed container;
• a method of reconstituting such lyophilisates ; and
• a stable, reconstituted, copanlisib-containing solution of increased solubility, suitable for further dilution and for therapeutic applications.

## Description

The present invention relates to :
- a method of preparing a stable, copanlisib-containing aqueous bulk solution suitable for lyophilisation and for therapeutic applications, (hereinafter referred to as "manufacturing process") ;
- a stable, copanlisib-containing bulk solution, (hereinafter referred to as "bulk solution"), of increased solubility which is directly suitable for lyophilisation ;
- a method of lyophilizing said bulk solution, (hereinafter referred to as "lyophilisation process") ;
- a stable, lyophilized, copanlisib-containing solid, in particular powder or cake, particularly containing a therapeutic dose of copanlisib in one or two containers, particularly one sealed container, (hereinafter referred to as "lyophilisate") ;
- a method of reconstituting said lyophilisate, (hereinafter referred to as "reconstitution method") ; and
- a stable, reconstituted, copanlisib-containing solution of increased solubility, suitable for further dilution and for therapeutic applications, (hereinafter referred to as "reconstituted solution").

Copanlisib is a novel intravenous pan-class I phosphatidylinositol-3-kinase (PI3K) inhibitor with kinase inhibitory activity predominantly against the PI3K-α and PI3K-δ isoforms, which are expressed in malignant B-cells. The PI3K pathway is involved in cell growth, survival and metabolism, and its dysregulation plays an important role in non-Hodgkin's lymphoma (NHL).

Copanlisib exhibits a broad spectrum of activity against tumors of multiple histologic types, both *in vitro* and *in vivo.*

Copanlisib may be synthesized according to the methods given in international patent application PCT/EP2003/010377, published as WO 04/029055 A1 on April 08, 2004, (which is incorporated herein by reference in its entirety), on pp. 26 *et seq*..

Copanlisib is published in international patent application PCT/US2007/024985, published as WO 2008/070150 A1 on June 12, 2008, (which is incorporated herein by reference in its entirety), as the compound of Example 13 : 2-amino-N-[7-methoxy-8-(3-morpholin-4-ylpropoxy)-2,3-dihydroimidazo[1,2-c]quinazolin-5-yl]pyrimidine-5-carboxamide, which is a free base. Copanlisib free base may be synthesized according to the methods given in WO 2008/070150, pp. 9 *et seq*., and on pp. 42 *et seq.* Biological test data for compounds of formula (I) is given in WO 2008/070150 on pp. 101 to 107.

Copanlisib dihydrochloride, which is 2-amino-N-[7-methoxy-8-(3-morpholin-4-ylpropoxy)-2,3-dihydroimid-azo[1,2-c]quinazolin-5-yl]pyrimidine-5-carboxamide dihydrochloride, (which is hereinafter referred to as "copanlisib dihydrochloride"), is published in international patent application PCT/EP2012/055600, published as WO 2012/136553 on October 11, 2012, (which is incorporated herein by reference in its entirety), as the compound of Examples 1 and 2 : 2-amino-N-[7-methoxy-8-(3-morpholin-4-ylpropoxy)-2,3-dihydroimidazo[1,2-c]quinazolin-5-yl]pyrimidine-5-carboxamide dihydrochloride : it may be synthesized according to the methods given in said Examples 1 and 2.

Copanlisib free base and copanlisib dihydrochloride can also be synthesized according to the methods published in international patent application PCT/EP2015/075789, published as WO 2016/071435 on May 12, 2016, (which is incorporated herein by reference in its entirety).

Copanlisib dihydrochloride exists in an amorphous form or in one of three crystalline forms, which are hydrate I, II and III :

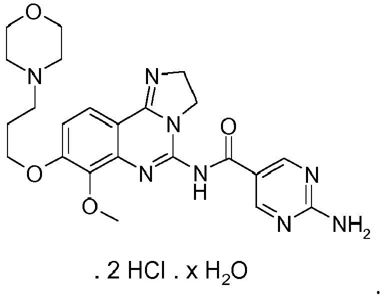

In the context of the present invention, unless otherwise specified, the term "copanlisib" or "drug substance" refers to copanlisib in the form of the free base, or of a salt, such as a hydrochloride, in particular a monohydrochloride or a dihydrochloride, or a tosylate, for example, it being possible for said form to exist as a hydrate, an anhydrate or as an amorphous form.

The compound used as drug substance in the present invention is copanlisib dihydrochloride.

### SPECIFIC TECHNICAL PROBLEMS ASSOCIATED WITH FORMULATING COPANLISIB

Copanlisib shows increasing solubility in aqueous solutions with decreasing pH which is, however, associated with decreasing stability and, additionally, a low local tolerability of a given potential formulation.

Copanlisib dihydrochloride shows a pH-dependent solubility : it is freely soluble in 0.1 M hydrochloric acid, phosphate buffer pH 1, phosphate buffer pH 2, citrate buffer pH 3. It is soluble in citrate buffer pH 4. It is very slightly soluble in citrate buffer pH 5. It is practically insoluble in phosphate buffer pH 6 and phosphate buffer pH 7.

According to the IUPAC definition, solubility is the analytical composition of a saturated solution expressed as a proportion of a designated solute in a designated solvent.

Solubility is the property of a solid, liquid, or gaseous chemical substance called solute to dissolve in a solid, liquid, or gaseous solvent. The solubility of a substance fundamentally depends on the physical and chemical properties of the solute and solvent as well as on temperature, pressure and the pH of the solution. The extent of the solubility of a substance in a specific solvent is measured as the saturation concentration, where adding more solute does not increase the concentration of the solution and begins to precipitate the excess amount of solute. The solubility of a substance is an entirely different property from the rate of solution, which is how fast it dissolves.

Under certain conditions, the equilibrium solubility can be exceeded to give a so-called supersaturated solution, which is metastable.

Supersaturated solutions will crystallize under specific conditions (see article *"*Crystallization of molecular systems from solution: phase diagrams, supersaturation and other basic concepts", Chemical Society Reviews, RSC Publishing. 43: 2286). In a normal solution, once the maximum amount of solute is dissolved, adding more solute would either cause the dissolved solute to precipitate out and/or for the solute to not dissolve at all (see article *"*Mechanism of precipitate formation during spontaneous crystallization from supersaturated aqueous solutions" ; Chem. Review. 83: 343-364. 2014). There are cases wherein solubility of a saturated solution is decreased by manipulating temperature, pressure, or volume but a supersaturated state does not occur. In these cases, the solute will simply precipitate out. This is because a supersaturated solution is in a higher energy state than a saturated solution.

Crucially, solubility applies to all areas of chemistry, geochemistry, inorganic, physical, organic and biochemistry. In all cases it will depend on the physical conditions (temperature, pressure and concentration) and the enthalpy and entropy directly relating to the solvents and solutes concerned.

Traditional lyophilisation is a complex process that requires a careful balancing of product, equipment, and processing techniques (see International Journal of Novel Trends in Pharmaceutical Sciences. 3(4). 2013). Besides various advantages, lyophilisation also bears many disadvantages such as long processing times, aseptic processing, limitations regarding size and filling volume of suitable containers and the related costs. As lyophilisation is a complex process, a certain experience and knowledge about critical formulation temperature/collapse temperature of the formulation and freeze-drying parameters is needed. Cake structure of the lyophilisate and solid state of active and inactive ingredients is affected by both composition and processing parameters.

In the specific case of copanlisib, the necessary therapeutic dose of copanlisib is only soluble in large volumes of diluent or at a pH which is not well tolerated for and after parenteral applications, i.e. during and after administration. Parenteral formulations should aim towards being isotonic and euhydric. Parenteral formulations should have a pH-value which is in accordance with the physiological pH-value at the site of application or, in case of infusions, with the physiological pH-value of the blood. Taking buffer capacity of human tissues, organs and blood into consideration, small-volume parenteral formulations with pH-values of 4-8 are regarded as well accepted.

As mentioned *supra*, copanlisib shows a pH-dependent solubility and a pH-dependent instability : copanlisib shows hydrolytic instability in various media such as water, buffer pH 7 and 0.1 M hydrochloric acid and 0.1 M sodium hydroxide solution.

In general, degradation of a given active pharmaceutical ingredient can take place due to chemical instability, resulting in a new chemical entity. For example, chemical instability can result from hydrolysis and/or oxidation. Degradation of active pharmaceutical ingredients is an undesired effect for pharmaceutical applications. The efficacy or availability of a drug can change dramatically.

An active pharmaceutical ingredient may also be physically unstable. Physical instability of a solution can result from crystallization and/or precipitation, when the solubility limit/solubility equilibrium of the active pharmaceutical ingredient is exceeded in a solution, which may result in particle formation. Particle formation in pharmaceuticals for parenteral application, in particular, makes the formulation harmful for clinical use.

Hence, copanlisib-containing bulk solutions used for further processing must show a sufficient physical and chemical stability ("holding time") to ensure the integrity of the formulation. Ready-to-use solutions must also show a sufficient physical and chemical stability (in-use stability) to avoid harm when finally administered to the patient.

As is known in the field of pharmaceutical formulation, lyophilisation, also known as freeze-drying, is a method of processing a liquid product into a dry solid product.

In general, lyophilisation is defined as a stabilizing process in which the product is frozen followed by elimination of the water content by sublimation.

The resulting lyophilised product should have an acceptable cake structure and sufficient stability ("shelf-life"), short rehydration/reconstitution time, and sufficient in-use- stability at the required temperature. One of the main disadvantages of lyophilized products is the fact that the stability of a drug depends on its physical state as well on the physical state of all components.

Prior to lyophilisation, a given active pharmaceutical ingredient, together with any excipients such as bulking agents, pH-adjusting agents, cryoprotectors which may be present, for example, must be dissolved in a suitable solvent to form a solution (a "bulk solution"). After freeze-drying, the resulting dry solid product and, with that, the active ingredient, must again be dissolved in a suitable diluent or solvent to form a solution (a "reconstituted solution"). This reconstituted solution can then be administered to the patient directly or after further dilution.

As a prerequisite for lyophilisation, a low filling volume of the vials is required in order to facilitate an efficient lyohilisation process. Therefore, lyophilisation is suitable only for active ingredients which allow for a high aqueous solubility or a low therapeutic dose in order to reduce the number of vials per administration. Thus, in case of high therapeutic doses, e.g. 60mg for copanlisib, the required number of vials per administration depend on the solubility of the drug substance.

Mannitol is widely used as bulking agent for lyophilized products. During lyophilisation, mannitol was shown to crystallize as three common stable anhydrous polymorphic forms (i.e. α, β and δ) or as mannitol hemihydrate (see *"*Mechanism of precipitate formation during spontaneous crystallization from supersaturated aqueous solutions" ; Chem. Review. 83: 343-364. 2014). The common stable anhydrous polymorphic forms of mannitol can also be named as modification I (hereinafter referred to as "Mod. I" or " β"), II (hereinafter referred to as "Mod. II" or "α ") and III (hereinafter referred to as "Mod. III" or "δ") (see Journal of Pharmaceutical Sciences Volume 89, Issue 4, April 2000, Pages 457-468. A. Burger, J.-O. Henck, S. Hetz, J. M. Rollinger, A. A. Weissnicht, H. Stöttner).

One of the reasons for the widespread use of mannitol is its tendency to crystallize from frozen aqueous solutions. This property should promote efficient freeze-drying and a physically stable, pharmaceutically elegant lyophilized solid.

Unlike many excipients (e.g., sorbitol and disaccharides), mannitol has a strong tendency to crystallize from a frozen aqueous solution, both during cooling and reheating. The vial breakage phenomenon is a striking illustration of this tendency. Mannitol has been observed to continue to crystallize after freeze-drying, especially as a result of heat and moisture, which indicates that the freeze-drying process can produce a partially amorphous and partially crystalline material (see Yu et al., Journal of Pharmaceutical Sciences Vol. 88, No. 2, February 1999*).*

There have been reports of adverse effects of mannitol on stability of drugs as lyophilized solids. Herman et al. reported that the rate of hydrolysis of methylprednisolone sodium succinate in the lyophilized solid state is significantly faster when mannitol is used as the bulking agent versus an amorphous excipient such as lactose (see Journal of Pharmaceutical Sciences Vol. 87, No. 8, August 1998*).* This instability of drug in the presence of mannitol was attributed at least in part to continued crystallization of mannitol from a system which is initially only partially crystalline. Although amorphous mannitol can serve as a stabiliser for the active pharmaceutical ingredient (API) (*see* Izutsu et al., Chemical and Pharmaceutical Bulletin Vol. 42, No. 1, January 1994*.* , the difficultly to maintain mannitol in the amorphous state during lyophilisation makes mannitol a poor choice as stabiliser (see Pikal, 2002. Freeze drying. In: Swarbrick, J., Boylan, J. (Eds.), Encyclopedia of Pharmaceutical Technology. Marcel Dekker, New York, pp. 1807-1833*).*

There is still a need for a better understanding of the physical chemistry of freeze-drying of mannitol-containing formulations in order to anticipate and avoid adverse effects of mannitol on physical and chemical stability of the lyophilized solid. A person skilled in the art should recognize that the physical state of mannitol in the lyophilized solid is affected by both formulation and processing parameters.

Given the foregoing, in view of the specific technical problems of formulating copanlisib, it is an object of this invention to provide :
- a pharmaceutically suitable formulation of copanlisib which prevents chemical and physical instability;
- a stable aqueous solution comprising copanlisib which is suitable for direct parenteral administration or further dilution ;
- a stable formulation comprising the total therapeutic dose of copanlisib in maximal 1 or 2 suitable containers ;
- a manufacturing process which allows for a pharmaceutically suitable formulation of copanlisib for parenteral administration.

It is to the Applicant's knowledge that such copanlisib-containing formulations, and such manufacturing processes for the manufacturing of copanlisib-containing formulations, are not known.

A pharmaceutically suitable formulation of copanlisib which prevents chemical and physical instability could be achieved by lyophilisation. An aqueous solution comprising copanlisib which is suitable for direct parenteral administration or further dilution requires a pH which is tolerated at the site of administration and does not distract the physiological pH of the blood. Regarding the low solubility of copanlisib at tolerated/physiological pH, lyophilisation of a large volume would be necessary to cover the total therapeutic dose. As the lyophilisation of large volume containers is not feasible, this approach would be expected by knowledge of prior art to result into lyophilisation of multiple containers to cover the therapeutic dose of copanlisib.

Therefore, an increased solubility of copanlisib in aqueous solution is required to allow for lyophilisation of a formulation comprising the total therapeutic dose in 1 or 2 containers.

In the light of the specific technical problems in formulating copanlisib, it was unexpectedly and surprisingly found, and this is the basis of the present invention, that a manufacturing process was achieved which results in an aqueous bulk solution of copanlisib which allows for increased drug solubility and is suitable for direct lyophilisation.

It was also surprisingly found that after reconstitution of the lyophilisate, a solution was achieved which shows increased copanlisib solubility and which is suitable direct application or dilution.

Further, the present invention is also based on the unexpected finding that the lyophilisate comprising copanlisib, a pH-adjusting agent and a bulking agent prevents chemical and physical instability during long term storage.

Specifically, it was surprisingly found that the manufacturing process according to this invention results in an aqueous copanlisib-containing bulk solution and that the reconstitution process even resulted in a copanlisib-containing reconstituted solution which shows an increased copanlisib solubility in the respective solvent which lies significantly above the expected solubility in that solvent according to the solubility data of copanlisib (see Examples section).

It was further surprisingly found that the copanlisib bulk solution and reconstituted solution does not show physical instability (e.g. precipitation) and allow for sufficient holding time and in-use-stability despite the metastable state of the solution : this is very unexpected in the light of prior art/general knowledge in the field.

It was further surprisingly found that the copanlisib bulk solution does not show crystallization/precipitation upon freezing and annealing during the lyophilisation process despite the metastable state of the solution : this too is very unexpected in the light of prior art/general knowledge in the field.

It was further surprisingly found that the lyophilisate formulation comprising copanlisib, a pH-adjusting agent and a crystalline bulking agent prevents crystallization of the amorphous drug substance phase on the crystalline bulking agent during long term storage : this is very unexpected in the light of prior art/general knowledge in the field due to the metastable state of the drug substance formulation.

It was further surprisingly found that the lyophilisate formulation comprising copanlisib, a pH-adjusting agent and a crystalline bulking agent prevents crystallization of the amorphous drug substance phase on the crystalline bulking agent at an increased residual moisture levels of the lyophilisate formulation : this is very unexpected in the light of prior art/general knowledge in the field due to the metastable state of the drug substance in the formulation.

As described above, a person skilled in the art would not come to the conclusion that the solubility of copanlisib can be enhanced and maintained for weeks as a bulk solution and as a reconstituted solution and during lyophilisation including freezing and annealing without solubilizing agents or stabilizing agents (e.g. emulsifiers, polymers).

As described above, a person skilled in the art would not come to the conclusion that the amorphous state of copanlisib achieved during lyophilisation can be physically maintained during long-term storage while mannitol is present in crystalline form.

Hence, the present invention relates to :
- a method of preparing a stable, copanlisib-containing aqueous bulk solution suitable for lyophilisation and for therapeutic applications, (hereinafter referred to as "manufacturing process") ;
- a stable, copanlisib-containing bulk solution, (hereinafter referred to as "bulk solution"), of increased solubility which is directly suitable for lyophilisation ;
- a method of lyophilizing said bulk solution, (hereinafter referred to as "lyophilisation process") ;
- a stable, lyophilized, copanlisib-containing solid, in particular powder or cake, particularly containing a therapeutic dose of copanlisib in one or two containers, particularly one sealed container, (hereinafter referred to as "lyophilisate") ;
- a method of reconstituting said lyophilisate, (hereinafter referred to as "reconstitution method") ; and
- a stable, reconstituted, copanlisib-containing solution of increased solubility, suitable for further dilution and for therapeutic applications, (hereinafter referred to as "reconstituted solution").

### DEFINITIONS USED IN THE PRESENT TEXT :

The term "pharmaceutical formulation" or "formulation" as used herein refers to a preparation which is in such form as to permit the biological activity of an active ingredient contained therein to be effective, and which contains no additional components which are unacceptably toxic to a subject to which the formulation would be administered.

The term "pH-adjusting agent" as used herein refers particularly but not exclusively to a buffered solution, which pH changes only marginally after addition of acidic or basic substances. Buffered solutions contain a mixture of a weak acid and its corresponding base, or a weak base and its corresponding acid, respectively. The term "buffering agent", as used herein, refers to a mixture of one or more of the aforementioned acids and bases. pH-adjusting agents which may be used in the present invention are, for example, buffers, such as citric acid or a salt thereof, acetic acid, or a salt thereof, or phosphoric acid or a salt thereof, or an inorganic acid, such as, for example, hydrochloric acid, boric acid, carbonic acid, bicarbonic acid, or an amino acid or an organic acid such as monocarbonic-, oxocarbonic or polycarbonic acid, or a base, such as, for example, sodium hydroxide, potassium hydroxide, sodium carbonate, sodium bicarbonate. In particular, the buffering agent is citric acid and NaOH. Most preferably the buffering agent comprises a mixture of citric acid and NaOH. In the context of the present invention, the term "citric acid" refers to the anhydrous form or a hydrate or a salt thereof such as sodium citrate, for example, unless otherwise specifically indicated.

The term "suitable diluent/solvent" as used herein refers particularly but not exclusively to aqueous sodium chloride solution 0.9%, glucose solution 5%, water for injection, ringer solution, mannitol 5%, etc..

The term "lyophilized" as used herein means that the composition has been lyophilized. During lyophilisation, the liquid formulation is frozen and the solutes are separated from the solvent. The solvent is removed by sublimation (i.e., primary drying) and next by desorption (i.e., secondary drying). Lyophilisation results in a cake or powder which can be stored over a long time period. Prior to administration, the lyophilized composition is reconstituted in a suitable solvent, particularly aqueous sodium chloride solution.

The term "reconstituted solution/formulation" as used herein refers to such a lyophilized composition after adding a suitable diluent.

Lyophilisation methods are well known in the art (e.g. see Wang, International Journal of Pharmaceutics Vol. 203, 2000*).* The lyophilized compositions of the present invention were prepared by various lyophilisation methods as described in the Examples section *infra.*

The term "fixed dose" as used herein refers to the administration of 60 mg of copanlisib.

In accordance with a first aspect, the present invention relates to a method of preparing a stable, copanlisib-containing aqueous bulk solution suitable for lyophilisation and for therapeutic applications, said bulk solution comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said bulk solution having a pH of between 4 and 5 (inclusive),
said method comprising :
- charging a suitable vessel with water for injection ;
- transferring copanlisib, in the form of a free base, or a salt, such as a hydrochloride, in particular a monohydrochloride or a dihydrochloride, or a tosylate, for example, to said vessel;
- allowing said copanlisib to dissolve, optionally by stirring for example, at a pH of between 2 and 3 (inclusive), thus forming a copanlisib-containing solution ;
- adding said buffering agent and said bulking agent to said copanlisib-containing solution;
- allowing said buffering agent and said bulking agent to dissolve, optionally by stirring for example;
- adjusting the pH of the thus-formed solution to a pH of 4 to 5 with an appropriate amount of sodium hydroxide solution, thus forming a bulk solution ;
- filtering said bulk solution in a sterile manner,
thus providing a stable, copanlisib-containing aqueous bulk solution suitable for direct lyophilisation and therapeutic applications ; and
- filling the thus-formed filtered bulk solution in one or more vials.

In accordance with an embodiment of the first aspect of the present invention, said copanlisib transferred to said vessel is copanlisib dihydrochloride.

Said method is hereinafter referred to as "manufacturing process" and said bulk solution is hereinafter referred to as "bulk solution".

Said manufacturing process provides a stable, copanlisib-containing aqueous bulk solution which gives increased solubility of copanlisib, *i.e.* which contains copanlisib at concentrations which are sufficiently high for their direct lyophilisation of the therapeutic dose in one or two vials, in particular one vial, which is of tolerated pH and which is suitable for therapeutic applications.

In accordance with a second aspect, the present invention relates to a stable, copanlisib-containing aqueous bulk solution of increased solubility which is directly suitable for lyophilisation, said bulk solution comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said bulk solution having a pH of between 4 and 5 (inclusive).

In accordance with an embodiment of the second aspect of the present invention, said therapeutic dose is 30, 45, 60 or 80 mg of copanlisib.

In accordance with an embodiment of the second aspect of the present invention, said therapeutic dose is 60 mg of copanlisib.

In accordance with an embodiment of the second aspect of the present invention, said pH-adjusting agent is a buffering agent.

In accordance with an embodiment of the second aspect of the present invention, said buffering agent is citric acid and sodium hydroxide.

In accordance with an embodiment of the second aspect of the present invention, said bulk copanlisib-containing solution contains citric acid in an amount of 0.18-18.5% w/w, particularly 0.18-3.7% w/w, in particular 0.37% w/w, of said bulk solution.

In accordance with an embodiment of the second aspect of the present invention, said bulk copanlisib-containing solution contains sodium hydroxide in an amount of 0-0.8% w/w, particularly 0.3-0.5% w/w, in particular 0.4 % w/w, of said bulk solution.

In accordance with an embodiment of the second aspect of the present invention, said bulk copanlisib-containing solution contains sodium hydroxide in an amount of 0.01-0.8% w/v, particularly 0.3-0.5 % w/v, in particular 0.4% w/v, of said bulk solution.

In accordance with an embodiment of the second aspect of the present invention, said bulking agent is mannitol.

In accordance with an embodiment of the second aspect of the present invention, said bulk copanlisib-containing solution contains mannitol in an amount of 3.0-23.1% w/w, particularly 3.0-15.4% w/w, in particular 7.7% w/w, of said bulk solution.

In accordance with an embodiment of the second aspect of the present invention, said bulk copanlisib-containing solution contains said copanlisib free base in an amount of 0.5-15.2% w/w, particularly 1.0-7.6% w/w, in particular 3.8% w/w, of said bulk solution.

In accordance with an embodiment of the second aspect of the present invention, in said bulk copanlisib-containing solution :
- said therapeutic dose of copanlisib is in an amount of 3.8% w/w of said bulk solution,
- said citric acid is in an amount of 0.37% w/w of said bulk solution,
- said sodium hydroxide is in an amount of 0.4 % w/w, of said bulk solution, and
- said mannitol is in an amount of 7.7% w/w, of said bulk solution,
said copanlisib-containing solution having a pH of between 4 and 5 (inclusive).

In accordance with an embodiment of the second aspect of the present invention, in said bulk copanlisib-containing solution :
- said therapeutic dose of copanlisib is in an amount of 68.4 mg,
- said citric acid is in an amount of 6.6 mg,
- said sodium hydroxide is in an amount of 7.2 mg, and
- said mannitol is in an amount of 136.8 mg,
said bulk copanlisib-containing solution having a pH of between 4 and 5 (inclusive),
said bulk copanlisib-containing solution having a volume of 1.71 ml.

In accordance with an embodiment of the second aspect of the present invention, said bulk copanlisib-containing solution contains copanlisib in an amount of 40 mg/ml.

In accordance with an embodiment of the second aspect of the present invention, said bulk copanlisib-containing solution contains 68.4 mg of copanlisib in a volume of 1.71 ml.

In accordance with an embodiment of the second aspect of the present invention, said bulk copanlisib-containing solution contains 60 mg of copanlisib in a volume of 1.5 ml.

In accordance with an embodiment of the second aspect of the present invention, said bulk copanlisib-containing solution is further diluted with a suitable diluent, such as sterile water for injection or sterile aqueous sodium chloride solution, particularly 0.9% aqueous sodium chloride solution, for example.

In accordance with an embodiment of the second aspect of the present invention, said bulk copanlisib-containing solution, wherein :
- said therapeutic dose of copanlisib is in an amount of 68.4 mg,
- said citric acid is in an amount of 6.6 mg,
- said sodium hydroxide is in an amount of 7.2 mg, and
- said mannitol is in an amount of 136.8 mg,
is further diluted with :
- a diluent, which is 0.9% aqueous sodium chloride solution, in an amount of 4.4 ml,
said bulk copanlisib-containing solution having a pH of between 4 and 5 (inclusive),
said bulk copanlisib-containing solution having a volume of 1.71 ml.

In accordance with an embodiment of the second aspect of the present invention, said bulk copanlisib-containing solution is further diluted with a suitable diluent, wherein said suitable diluent is 0.9% aqueous sodium chloride solution, particularly in a volume of 100 ml.

In accordance with an embodiment of the second aspect of the present invention, said bulk copanlisib-containing solution is further diluted with a suitable diluent, wherein said suitable diluent is 0.9% aqueous sodium chloride solution, particularly in a volume of 100 ml, which is contained in an infusion bag, particularly made of polyethylene (PE), polypropylene (PP), polyvinylchloride (PVC), or ethylene vinyl acetate (EVA).

Said bulk solution is hereinafter referred to as such.

In accordance with a third aspect, the present invention relates to a method of lyophilizing a stable, copanlisib-containing aqueous bulk solution suitable for lyophilisation and for therapeutic applications, said composition comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said bulk solution having a pH of between 4 and 5 (inclusive),
said lyophilisation method including : freezing, annealing, primary and secondary drying steps;
said method thus providing a stable, lyophilized, copanlisib-containing solid, in particular powder or cake, suitable for dilution and for therapeutic applications, said solid, in particular powder or cake, comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example.

Said stable, lyophilized, copanlisib-containing solid, in particular powder or cake, once reconstituted into a reconstituted solution, is suitable for further dilution and for therapeutic applications.

Said method is hereinafter referred to as "lyophilisation process". Said lyophilisation process provides a stable, lyophilized, copanlisib-containing solid, in particular powder or cake, which has acceptable cake structure and short reconstitution time.

In accordance with a fourth aspect, the present invention relates to a stable, lyophilized, copanlisib-containing solid, in particular powder or cake, suitable for dilution and for therapeutic applications, said solid, in particular powder or cake, comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example.

In accordance with an embodiment of the fourth aspect of the present invention, wherein said therapeutic dose is 30, 45, 60 or 80 mg of copanlisib.

In accordance with an embodiment of the fourth aspect of the present invention, wherein said therapeutic dose is 60 mg of copanlisib.

In accordance with an embodiment of the fourth aspect of the present invention, said pH-adjusting agent is a buffering agent.

In accordance with an embodiment of the fourth aspect of the present invention, said buffering agent is citric acid and sodium hydroxide.

In accordance with an embodiment of the fourth aspect of the present invention, said buffering agent is a citric buffer, which gives a pH value of between 4 to 5 (inclusive), in particular of 5, when reconstituted with a suitable diluent, such as sterile water for injection or sterile aqueous sodium chloride solution, particularly 0.9% aqueous sodium chloride solution, for example.

In accordance with an embodiment of the fourth aspect of the present invention, wherein said lyophilized, copanlisib-containing solid, in particular powder or cake, contains citric acid in an amount of 0.7- 75 % w/w, particularly 1.5-30% w/w, in particular 2.98% w/w, of said lyophilized, copanlisib-containing solid.

In accordance with an embodiment of the fourth aspect of the present invention, wherein said lyophilized, copanlisib-containing solid, in particular powder or cake, contains sodium hydroxide in an amount of 0-6% w/w, particularly 3.3-4.0 % w/w, in particular 3.7% w/w, of said lyophilized, copanlisib-containing solid.

In accordance with an embodiment of the fourth aspect of the present invention, wherein said lyophilized, copanlisib-containing solid, in particular powder or cake, contains sodium hydroxide in an amount of 0.01-6% w/w, particularly 3.3-4.0 % w/w, in particular 3.7% w/w, of said lyophilized, copanlisib-containing solid.

In accordance with an embodiment of the fourth aspect of the present invention, wherein said bulking agent is mannitol.

In accordance with an embodiment of the fourth aspect of the present invention, wherein said lyophilized, copanlisib-containing solid, in particular powder or cake, contains mannitol in an amount of 15.4-82.1% w/w, particularly 30.8-82.1% w/w, in particular 61.6% w/w, of said lyophilized, copanlisib-containing solid.

In accordance with an embodiment of the fourth aspect of the present invention, wherein said lyophilized, copanlisib-containing solid, in particular powder or cake, contains said copanlisib free base in an amount of 7.7-92.4% w/w, particularly 7.7-61.6% w/w, in particular 30.8% w/w, of lyophilized, copanlisib-containing solid.

In accordance with an embodiment of the fourth aspect of the present invention, wherein in said lyophilized, copanlisib-containing solid, in particular powder or cake :
- said therapeutic dose of copanlisib is in an amount of 30.8% w/w of said lyophilized, copanlisib-containing solid,
- said citric acid is in an amount of 2.98% w/w of said lyophilized, copanlisib-containing solid,
- said sodium hydroxide is in an amount of 3.7% w/w of said lyophilized, copanlisib-containing solid, and
- said mannitol is in an amount of 61.6% w/w, of said lyophilized, copanlisib-containing solid,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

In accordance with an embodiment of the fourth aspect of the present invention, in said lyophilized, copanlisib-containing solid, in particular powder or cake :
- said therapeutic dose of copanlisib is in an amount of 30, 45 or 60 mg,
- said citric acid is in an amount of 5.8 mg,
- said sodium hydroxide is in an amount of 6.3 mg, and
- said mannitol is in an amount of 120 mg,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

In accordance with an embodiment of the fourth aspect of the present invention, in said lyophilized, copanlisib-containing solid, in particular powder or cake :
- said therapeutic dose of copanlisib is in an amount of 80 mg,
- said citric acid is in an amount of 7.7 mg,
- said sodium hydroxide is in an amount of 8.4 mg, and
- said mannitol is in an amount of 160 mg,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

In accordance with an embodiment of the fourth aspect of the present invention, said lyophilized, copanlisib-containing solid, in particular powder or cake, is contained in one container, particularly a sealed container, particularly an injection vial, more particularly a 6 ml injection vial.

In accordance with an embodiment of the fourth aspect of the present invention, said lyophilized, copanlisib-containing solid, in particular powder or cake, contains copanlisib in an amount of 68.4 mg.

Said solid, in particular powder or cake, is hereinafter referred to as "lyophilisate".

Said stable, lyophilized, copanlisib-containing solid, in particular powder or cake, contains copanlisib in an amorphous solid state which shows sufficient stability.

Further, said stable, lyophilized, copanlisib-containing solid, in particular powder or cake, allows for long term storage as lyophilized composition, *i.e.* has a long shelf life, and, once reconstituted as a stable, reconstituted, copanlisib-containing solution, has sufficient in-use-time after reconstitution.

Hence, said stable, lyophilized, copanlisib-containing solid, in particular powder or cake, once reconstituted into a reconstituted solution, is suitable for direct therapeutic applications, or for further dilution with a suitable diluent, such as sterile water for injection or sterile aqueous sodium chloride solution, particularly 0.9% aqueous sodium chloride solution, for example, and, in turn, for further therapeutic applications.

In order to obtain physical stability during and after the lyophilisation process the inventive liquid composition comprises a bulking agent. Bulking agents typically are used in the art to provide structure and weight to the "cake" produced as a result of lyophilisation. Any suitable bulking agent known in the art may be used in connection with the inventive lyophilized composition. Suitable bulking agents include, for example, mannitol, dextran, cyclodextrines and glycine, trehalose, saccharose.

In accordance with a fifth aspect, the present invention relates to a method of reconstituting a stable, lyophilized, copanlisib-containing solid, in particular powder or cake, suitable for dilution and for therapeutic applications, said stable, lyophilized solid, in particular powder, comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example,
said reconstitution method comprising :
- adding, to said stable, lyophilized solid, in particular powder, in a suitable container, particularly a sealed container, particularly an injection vial, more particularly a 6 ml injection vial, a diluent, such as sterile water for injection or sterile aqueous sodium chloride solution, particularly 0.9% aqueous sodium chloride solution, for example ;
said method thus providing a stable, reconstituted, copanlisib-containing solution, suitable for further dilution and for therapeutic applications, said reconstituted solution comprising :
- copanlisib, particularly in an amount suitable as a therapeutic dose,
- one or more pH-adjusting agents, particularly a buffering agent which in particular comprises a weak acid, such as citric acid for example, and a strong base, such as sodium hydroxide, for example, and
- a bulking agent,
said reconstituted solution having a pH of between 4 and 5 (inclusive).

In accordance with an embodiment of the fifth aspect of the present invention, said therapeutic dose is 30, 45, 60 or 80 mg of copanlisib.

In accordance with an embodiment of the fifth aspect of the present invention, said therapeutic dose is 60 mg of copanlisib.

In accordance with an embodiment of the fifth aspect of the present invention, said pH-adjusting agent is a buffering agent.

In accordance with an embodiment of the fifth aspect of the present invention, said buffering agent is citric acid and sodium hydroxide.

In accordance with an embodiment of the fifth aspect of the present invention, wherein said reconstituted copanlisib-containing solution produced by said method contains citric acid in an amount of 0.08 - 15.0% w/v, particularly 0.15-3.0% w/v, in particular 0.15% w/v, of said reconstituted solution.

In accordance with an embodiment of the fifth aspect of the present invention, wherein said reconstituted copanlisib-containing solution produced by said method contains sodium hydroxide in an amount of 0-0.3% w/v, particularly 0.14-0.18 % w/v, in particular 0.16 % w/v, of said reconstituted solution.

In accordance with an embodiment of the fifth aspect of the present invention, wherein said reconstituted copanlisib-containing solution produced by said method contains sodium hydroxide in an amount of 0.01-0.3% w/v, particularly 0.14-0.18 % w/v, in particular 0.16 % w/v, of said reconstituted solution.

In accordance with an embodiment of the fifth aspect of the present invention, wherein said bulking agent is mannitol.

In accordance with an embodiment of the fifth aspect of the present invention, wherein said reconstituted copanlisib-containing solution produced from said method contains mannitol in an amount of 1.5-24.0% w/v, particularly 1.5-16.0% w/v, in particular 3.0% w/v, of said reconstituted solution.

In accordance with an embodiment of the fifth aspect of the present invention, wherein said diluent is sterile aqueous sodium chloride solution, particularly 0.9% aqueous sodium chloride solution.

In accordance with an embodiment of the fifth aspect of the present invention, wherein said reconstituted copanlisib-containing solution produced therefrom contains said copanlisib free base in an amount of 0.5-16.0% w/v, particularly 1.0-8.0% w/v, in particular 1.5% w/v, of said reconstituted solution.

In accordance with an embodiment of the fifth aspect of the present invention, in said reconstituted copanlisib-containing solution produced from said method :
- said therapeutic dose of copanlisib is in an amount of 1.5% w/v of said reconstituted solution,
- said citric acid is in an amount of 0.15% w/v of said reconstituted solution,
- said sodium hydroxide is in an amount of 0.16 % w/v, of said reconstituted solution, and
- said mannitol is in an amount of 3.0% w/v, of said reconstituted solution,
said diluent being 0.9% aqueous sodium chloride solution,
said copanlisib-containing solution having a pH of between 4 and 5 (inclusive).

In accordance with an embodiment of the fifth aspect of the present invention, said reconstituted copanlisib-containing solution produced from said method contains copanlisib in an amount of 15 mg/ml.

In accordance with an embodiment of the fifth aspect of the present invention, said reconstituted copanlisib-containing solution produced from said method contains 68.4 mg of copanlisib in a volume of 4.55 ml.

In accordance with an embodiment of the fifth aspect of the present invention, said reconstituted copanlisib-containing solution produced from said method contains 60 mg of copanlisib in a volume of 4 ml.

In accordance with an embodiment of the fifth aspect of the present invention, in said reconstituted copanlisib-containing solution produced from said method :
- said therapeutic dose of copanlisib is in an amount of 68.4 mg,
- said citric acid is in an amount of 6.6 mg,
- said sodium hydroxide is in an amount of 7.2 mg, and
- said mannitol is in an amount of 136.8 mg,
- said diluent, which is 0.9% aqueous sodium chloride solution, is in an amount of 4.4ml,
said reconstituted copanlisib-containing solution having a pH of between 4 and 5 (inclusive),
said reconstituted copanlisib-containing solution having a volume of 4.55 ml.

In accordance with an embodiment of the fifth aspect of the present invention, said reconstituted copanlisib-containing solution produced from said method is contained in one container, particularly a sealed container, particularly an injection vial, more particularly a 6 ml injection vial.

Said stable, reconstituted, copanlisib-containing solution is of tolerated pH and which is suitable for direct therapeutic applications and/or for further dilution with a suitable diluent, such as sterile water for injection or sterile aqueous sodium chloride solution, particularly 0.9% aqueous sodium chloride solution, for example, and, in turn, for further therapeutic applications.

In accordance with an embodiment of the fifth aspect of the present invention, said reconstituted copanlisib-containing solution produced from said method is further diluted with a suitable diluent, such as sterile water for injection or sterile aqueous sodium chloride solution, particularly 0.9% aqueous sodium chloride solution, for example.

In accordance with an embodiment of the fifth aspect of the present invention, said suitable diluent is 0.9% aqueous sodium chloride solution, particularly in a volume of 100 ml.

In accordance with an embodiment of the fifth aspect of the present invention, said reconstituted copanlisib-containing solution produced from said method is contained in an infusion bag, particularly made of polyethylene (PE), polypropylene (PP), polyvinylchloride (PVC), or ethylene vinyl acetate (EVA).

Said method is hereinafter referred to as "reconstitution method".

In accordance with a sixth aspect, the present invention relates to a stable, reconstituted, copanlisib-containing solution of increased solubility, suitable for further dilution and for therapeutic applications, said reconstituted solution comprising :
- copanlisib, particularly in an amount suitable as a therapeutic dose, in one or two containers, particularly one sealed container,
- one or more pH-adjusting agents, particularly a buffering agent which in particular comprises a weak acid, such as citric acid for example, and a strong base, such as sodium hydroxide, for example, and
- a bulking agent,
said reconstituted solution having been prepared with a suitable diluent, such as sterile water for injection or sterile aqueous sodium chloride solution, particularly 0.9% aqueous sodium chloride solution, for example,
said reconstituted solution having a pH of between 4 and 5 (inclusive).

In accordance with an embodiment of the sixth aspect of the present invention, said therapeutic dose is 30, 45, 60 or 80 mg of copanlisib.

In accordance with an embodiment of the sixth aspect of the present invention, said therapeutic dose is 60 mg of copanlisib.

In accordance with an embodiment of the sixth aspect of the present invention, wherein said pH-adjusting agent is a buffering agent.

In accordance with an embodiment of the sixth aspect of the present invention, wherein said buffering agent is citric acid and sodium hydroxide.

In accordance with an embodiment of the sixth aspect of the present invention, wherein said reconstituted copanlisib-containing solution contains citric acid in an amount of 0.08 - 15.0% w/v, particularly 0.15-3.0% w/v, in particular 0.15% w/v, of said reconstituted solution.

In accordance with an embodiment of the sixth aspect of the present invention, said reconstituted copanlisib-containing solution contains sodium hydroxide in an amount of 0-0.3% w/v, particularly 0.14-0.18 % w/v, in particular 0.16 % w/v, of said reconstituted solution.

In accordance with an embodiment of the sixth aspect of the present invention, said reconstituted copanlisib-containing solution contains sodium hydroxide in an amount of 0.01-0.3% w/v, particularly 0.14-0.18 % w/v, in particular 0.16 % w/v, of said reconstituted solution.

In accordance with an embodiment of the sixth aspect of the present invention, wherein said bulking agent is mannitol.

In accordance with an embodiment of the sixth aspect of the present invention, wherein said reconstituted copanlisib-containing solution which contains mannitol in an amount of 1.5-24.0% w/v, particularly 1.5-16.0% w/v, in particular 3.0% w/v, of said reconstituted solution.

In accordance with an embodiment of the sixth aspect of the present invention, wherein said diluent is sterile aqueous sodium chloride solution, particularly 0.9% aqueous sodium chloride solution.

In accordance with an embodiment of the sixth aspect of the present invention, wherein said reconstituted copanlisib-containing solution contains said copanlisib free base in an amount of 0.5-16.0% w/v, particularly 1.0-8.0% w/v, in particular 1.5% w/v, of said reconstituted solution.

In accordance with an embodiment of the sixth aspect of the present invention, wherein in said reconstituted copanlisib-containing solution :
- said therapeutic dose of copanlisib is in an amount of 1.5% w/v of said reconstituted solution,
- said citric acid is in an amount of 0.15% w/v of said reconstituted solution,
- said sodium hydroxide is in an amount of 0.16 % w/v, of said reconstituted solution, and
- said mannitol is in an amount of 3.0% w/v, of said reconstituted solution,
said diluent being 0.9% aqueous sodium chloride solution,
said copanlisib-containing solution having a pH of between 4 and 5 (inclusive).

In accordance with an embodiment of the sixth aspect of the present invention, wherein in said reconstituted copanlisib-containing solution :
- said therapeutic dose of copanlisib is in an amount of 68.4 mg,
- said citric acid is in an amount of 6.6 mg,
- said sodium hydroxide is in an amount of 7.2 mg, and
- said mannitol is in an amount of 136.8 mg,
- said diluent, which is 0.9% aqueous sodium chloride solution, is in an amount of 4.4ml,
said reconstituted copanlisib-containing solution having a pH of between 4 and 5 (inclusive),
said reconstituted copanlisib-containing solution having a volume of 4.55 ml.

In accordance with an embodiment of the sixth aspect of the present invention, wherein said reconstituted copanlisib-containing solution is contained in one container, particularly a sealed container, particularly an injection vial, more particularly a 6 ml injection vial.

In accordance with an embodiment of the sixth aspect of the present invention, wherein said reconstituted copanlisib-containing solution contains copanlisib in an amount of 15 mg/ml.

In accordance with an embodiment of the sixth aspect of the present invention, said reconstituted copanlisib-containing solution contains 60 mg of copanlisib, particularly in a volume of 4 ml.

In accordance with an embodiment of the sixth aspect of the present invention, said reconstituted copanlisib-containing solution is further diluted with a suitable diluent, such as sterile water for injection or sterile aqueous sodium chloride solution, particularly 0.9% aqueous sodium chloride solution, for example.

In accordance with an embodiment of the sixth aspect of the present invention, wherein said suitable diluent is 0.9% aqueous sodium chloride solution, particularly in a volume of 100 ml.

In accordance with an embodiment of the sixth aspect of the present invention, said reconstituted copanlisib-containing solution is contained in an infusion bag, particularly made of polyethylene (PE), polypropylene (PP), polyvinylchloride (PVC), or ethylene vinyl acetate (EVA).

Said stable, reconstituted, copanlisib-containing solution contains copanlisib at concentrations which are sufficiently high, *i.e.* that copanlisib has increased solubility, and is of tolerated pH for their direct therapeutic applications and/or for further dilution with a suitable diluent, such as sterile water for injection or sterile aqueous sodium chloride solution, particularly 0.9% aqueous sodium chloride solution, for example, and, in turn, for further therapeutic applications. Said stable, reconstituted, copanlisib-containing solution allows for sufficient in-use-time-after having been reconstituted.

Said reconstituted solution is hereinafter referred to as such.

### EXPERIMENTAL SECTION

### EXAMPLE 1 : Method of preparing a stable, copanlisib-containing aqueous bulk solution suitable for lyophilisation and for therapeutic applications.

Said method is hereinafter referred to as "manufacturing process" and the bulk solution thus produced is hereinafter referred to as "bulk solution".

The manufacturing process of copanlisib bulk solution is conducted as follows:
The manufacturing vessel is charged with water for injection. Copanlisib is transferred to the vessel and dissolved while stirring at pH 2-3. Bulking agent and buffering agent is added and dissolved while stirring. The pH of the solution is adjusted to pH 5.0 with an appropriate amount of sodium hydroxide solution. Afterwards, a sterile filtration of the bulk solution is performed and the solution is filled into vials.

### Solubility:

**Table 1: Solubility of copanlisib dihydrochloride**

| **Solvent** | **Solubility at 25 °C [mg/mL]** | **Descriptive term ^{a}** |
|---|---|---|
| Water | 2.3· 10² | freely soluble |
| 0.1 M Hydrochloric acid | 1.9·10² | freely soluble |
| Buffer (phosphate) pH 1 | 5.1· 10² | freely soluble |
| Buffer (phosphate) pH 2 | 2.5· 10² | freely soluble |
| Buffer (citrate) pH 3 | 2.5· 10² | freely soluble |
| Buffer (citrate) pH 4 | 2.5·10¹ | soluble |
| Buffer (citrate) pH 5 | 6.2·10⁻¹ | very slightly soluble |
| Buffer (phosphate) pH 6 | 1·10⁻³ | practically insoluble |
| Buffer (phosphate) pH 7 | < 110⁻³ | practically insoluble |
| a according to Ph. Eur./USP | | |

### Chemical stability in solution

The different aqueous solutions (0.05 % copanlisib; after addition of 50 % 2-propanol, [buffer solution filtered with 0.5µm membrane filter]) were stored at 25°C and 70°C for 24 h and one week. An analytical HPLC-method has been developed to determine assay and organic impurities of copanlisib as well as degradation products.

**Table 2 : Results on hydrolytic stability of copanlisib**

| **Conditions** | **Appearance** | **Sum of Degradation products [area-%]** | **Degrad. Products max. single [area-%]** |
|---|---|---|---|
| **Water:** | | | |
| Initial | slightly colored solution | 2.79 | 0.25 |
| 24 h, 25°C | slightly colored solution | 3.43 | 0.23 |
| 24h, 70°C | slightly colored solution | 58.00 | 25.89 |
| 1 week, 25°C | slightly colored solution | 5.33 | 0.54 |
| 1 week, 70°C | slightly colored solution | 98.59 | 45.44 |

| **Buffer pH 7:** | | | |
|---|---|---|---|
| Initial | slightly colored turbid solution | 3.15 | 0.23 |
| 24 h, 25°C | slightly colored turbid solution | 3.22 | 0.20 |
| 24 h, 70°C | slightly colored solution | 56.06 | 23.25 |
| 1 week, 25°C | slightly colored turbid solution | 4.85 | 0.82 |
| 1 week, 70°C | slightly colored solution | 97.65 | 39.01 |

| **0.1M HCl:** | | | |
|---|---|---|---|
| Initial | slightly colored solution | 5.87 | 1.13 |
| 24 h, 25°C | slightly colored solution | 8.75 | 1.90 |
| 24h, 70°C | slightly colored solution | 92.49 | 22.82 |
| 1 week, 25°C | slightly colored solution | 24.27 | 7.15 |
| 1 week, 70°C | slightly colored solution | 100.00 | 25.48 |

| **0.1 M NaOH:** | | | |
|---|---|---|---|
| Initial | slightly colored solution | 30.72 | 6.51 |
| 24 h, 25°C | slightly colored solution | 45.40 | 10.02 |
| 24h, 70°C | slightly colored solution | 99.88 | 23.94 |
| 1week, 25°C | slightly colored solution | 86.64 | 22.03 |
| 1week, 70°C | slightly colored solution | 99.90 | 32.63 |

### Physical stability

With regard to the required holding time of the bulk solution during the manufacturing process including filling of vials and freezing of the solution, chemical and physical stability in solution is of integral importance even for the development of a lyophilisate.

Reconstituted solutions must also show a sufficient physical and chemical stability (in-use stability) to avoid harm when finally administered to the patient.

The chemical stability of the copanlisib bulk solution increases with increasing pH. Especially the formation of the main degradation product aminoethyltrioxoquinazolinpyramide (hereinafter abbreviated and referred to as "AETQP") revealed clear pH dependency in the investigated range of pH 3 to pH 6 showing higher stability at higher pH.

However, at pH 6 precipitation of the copanlisib dihydrochloride bulk solution was visually detectable under stress conditions. Therefore, the pH of the bulk solution needed to be below pH 6.

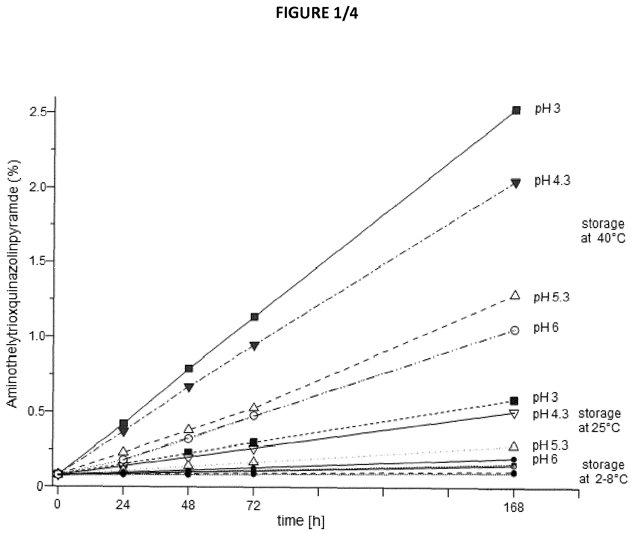

Figure 1/4 shows the pH-dependent formation of AETQP in copanlisib bulk solution at 2-8 °C, 25 °C and 40 °C.

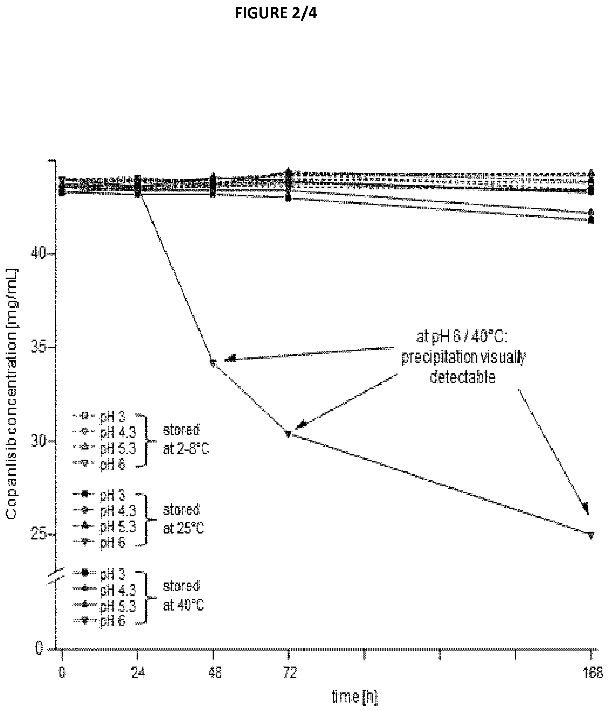

Figure 2/4 shows the pH-dependent stability of copanlisib bulk solution.

### Bulk solution (40mg/ml, copanlisib free base)

Investigations regarding the physical stability of the copanlisib bulk solution were performed after filling into vials. Visual inspection were performed for the vials filled with bulk solution approximately 17 days after start of manufacturing and storage at 2-8°C without any findings. Further investigations were performed later on, approximately 3 weeks after - manufacturing of the bulk solution (Table 3). No precipitation was observed within the test period of additional 14 days at room temperature or storage at 2-8°C. Thus, the copanlisib bulk solution manufactured under the described conditions including sterile filtration and filling into glass vials revealed no precipitation tendency within 5 weeks after manufacturing.

**Table 3: Effect of temperature on physical stability of bulk solution in vials (n=3), investigations started approx. 3 weeks after manufacturing and storage at 2-8°C (holding time of bulk solution) :**

| **Observations** | **20°C** | **4°C** |
|---|---|---|
| **Start (3 weeks after manufacturing)** | clear solution | clear solution |
| **24 h** | clear solution | clear solution |
| **48 h** | clear solution | clear solution |
| **5 days** | clear solution | clear solution |
| **7 days** | clear solution | clear solution |
| **14 days** | clear solution | clear solution |

### EXAMPLE 2 : Method of lyophilizing a stable, copanlisib-containing aqueous bulk solution suitable for lyophilisation and for therapeutic applications.

Said method is hereinafter referred to as "lyophilisation process", the lyophilized solid, in particular powder or cake, thus produced, is hereinafter referred to as "lyophilisate" and the stable, lyophilized, copanlisib-containing solid, in particular powder or cake, suitable for therapeutic applications, comprising, *inter alia*, a therapeutic dose of copanlisib in one or two containers, particularly one sealed containers is hereinafter referred to as "ready-to-reconstitute lyophilisate" or "drug product".

### Lyophilisation process

Lyophilisation or freeze drying is a well-known stabilization process for liquid formulations. A typical lyophilisation process can be divided into three stages: freezing including annealing, primary drying, and secondary drying.

Initial freezing of the liquid formulation leads to a conversion of water into ice and crystallization of the solutes. An additional annealing step ensures complete crystallization of the solutes. During primary dying the frozen ice sublimates under vacuum. Remaining moisture is evaporated from the matrix during secondary drying. Finally, a dried and solid cake is obtained comprising the solutes of the initial solution.

The lyophilisation process was developed in lab scale (HOF, Fabrik-Nr.: Com. 41239). For production of lyophilisate in in pilot and commercial scale HOF, Fabrik-Nr.: Com. 41041 was used so far.

**Table 4a: Nominal filling and filling including overfill and amount of copanlisib free base and copanlisib dihydrochloride for a 60 mg dose**

| | **Nominal filling** | **Filling including overfill** |
|---|---|---|
| Copanlisib free base | 60 mg | 68.4 mg |
| Copanlisib dihydrochloride | 69.1mg | 78.8 mg |
| Volume of bulk solution/ vial | 1.5 mL | 1.71 mL |
| Volume of reconst. solution /vial | 4.0 mL | 4.55 mL |

The freeze drying parameters are as follows:

**Table 4b: Comparison of freeze drying processes applied for copanlisib lyophilisates**

| | **Freeze drying process applied to copanlisib lyophilisate 80 mg** | **Initial freeze drying process applied to copanlisib lyophilisate 60 mg** | **Final freeze drying process for copanlisib lyophilisate 60 mg** |
|---|---|---|---|
| Filling of bulk solution | nominal filling 2.0 mL plus additional 0.1 mL overfill | nominal filling 1.5 mL plus additional 0.07 mL or 0.21 mL overfill | nominal filling 1.5 mL plus additional 0.21mL overfill |
| Freezing | Ramp to -45 °C, hold for 2 h | Ramp to -45 °C, hold for 1.5 h | Ramp to -45 °C, hold for 1.5 h |
| Annealing | -20 °C for 4 h | -20 °C for 2 h | -20 °C for 3.5 h |
| Primary Drying | Isothermal hold at -5 °C/ 100 µbar for 23 h | Drying Ramp from -45 °C to +30 °C with 0.08 °C/min / 100 µbar, duration 15.6 h | Isothermal hold at +5 °C / 110 µbar for 14 h |
| Secondary Drying | Ramp to +40 °C, hold for 8 h | Ramp to +35 °C, hold for 6.2 h | Ramp to +35 °C, hold for 4 h |

**Table 5: Final process including ranges:**

| **Step** | **Temperature** | **Pressure** | **Time** |
|---|---|---|---|
| Freezing | - 42 to -48 °C | Atmospheric | ≥ 1.5 h |
| Annealing | -18 to -24 °C | Atmospheric | ≥ 3.0 h |
| Primary drying | 1-9 °C | 70-150 µbar | ≥ 10 h |
| Secondary drying | 30-40 °C | 70-150 µbar | 3-8 h |
| Storage until unloading | 5-25 °C | 70-150 µbar | < 72 h |

### Determination of solubility:

The solubility of copanlisib dihydrochloride was determined using the flask method by stirring copanlisib in the solvents to be tested in a thermostatically controlled water bath at 25 °C for 19 hours. The suspension was centrifuged before HPLC analysis. The concentration was monitored by HPLC against external standard.

### Determination of pH :

The pH of the Copanlisib solutions are determined potentiometric according to Ph. Eur.

### Storage stability experiment:

Throughout the development of copanlisib lyophilisates, analysis of the physicochemical state of the lyophilized product confirmed only crystalline peaks based on mannitol (present in modification III) and showed no indication of crystallization events of the drug substance. Stress testing of technical and clinical batches revealed that this behaviour is maintained for at least 18 months of storage at 30 °C/75 % RH.

**Table 6: Solid state of copanlisib lyophilisate 60 mg for injection after stress testing**

| **Batch no.** | **Storage conditions** | **Solid state (XRPD)** |
|---|---|---|
| Batch A | 18 months, 30 °C/75 % RH | Mannitol Mod. III, drug substance amorphous |
| Batch B | 18 months, 30 °C/75 % RH | Mannitol Mod. III, drug substance amorphous |
| Batch C | 12 months, 30 °C/75 % RH | Mannitol Mod. III, drug substance amorphous |

Batch A, B and C have the following amounts of components as shown below in Table 6a.

**Table 6a: Amounts of components in Batches A, B and C of copanlisib lyophilisate 60 mg**

| | Lyophilisate (%, w/w) |
|---|---|
| Copanlisib (free base) | 31 |
| Mannitol | 62 |
| Citric acid | 3 |

Sodium hydroxide which is needed for pH-adjustment is contained q.s.

There are also drug product batches of the 80 mg dose strength that were stored at the intended storage condition of 2 - 8 °C as well as at elevated conditions of 25 °C/60 % RH which confirmed that the amorphous state of the drug substance was maintained even over 4 years of storage ( Table 7).

**Table 7: Solid state of copanlisib lyophilisate 80 mg for injection after long term testing**

| **Batch no.** | **Storage conditions** | **Solid state (XRPD)** |
|---|---|---|
| Batch D | 48 months, 2-8°C | Mannitol Mod. III, drug substance amorphous |
| Batch D | 48 months, 25°C/60 % RH | Mannitol Mod. III, drug substance amorphous |
| Batch E | 37 months, 2-8°C | Mannitol Mod. III, drug substance amorphous |
| Batch E | 37 months, 25°C/60 % RH | Mannitol Mod. III, drug substance amorphous |

Thus, it can be confirmed that the amorphous state of the drug substance which ensures rapid dissolution upon reconstitution is preserved throughout the product shelf life of the product.

Furthermore, the residual moisture of the lyophilisate was monitored during long term stability studies. The results for the copanlisib lyophilisate 60 mg and 80 mg formulation are displayed in the following tables. Regarding all investigated batches and conditions the water content in the lyophilisates was within the specified limit of 2.0%. Solid state investigations (XRPD) of the lyophilisates confirmed the amorphous state of the drug substance independent of the water content investigated. Moreover, mannitol is still present in modification III.

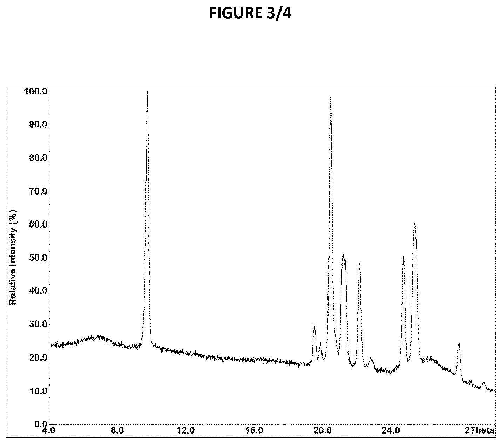

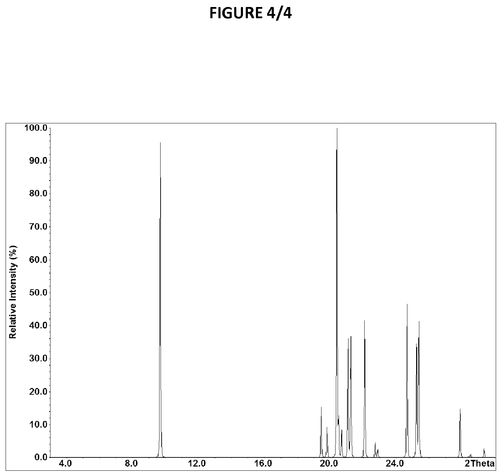

Figure 3/4 shows an X-ray powder diffractogram of copanlisib 60 mg for injection after 18 Months at 30 °C and 75% relative humidity (Batch A see Table 6a).

Table 7a shows the corresponding 2θ Values of the diffraction peaks.

As reference, the X-ray powder diffractogram of D-Mannitol Modification III (also known as the δ form) is shown in Figure 4/4. The diffractogram was simulated from the single crystal X-ray structure published by Botez et al. 2003 (see C.E. Botez, P. W. Stephens, C. Nunes, R. Suryanarayanan, Powder Diffr. (2003), 18, 214).

**Table 7a: 2θ Values of the diffraction peaks of copanlisib 60 mg for injection after 18 months storage at 30 °C and 75% relative humidity (Batch A see Table 6a)**

| Diffraction angle (2θ, °) |
|---|
| 9.7 |
| 19.4 |
| 19.8 |
| 20.4 |
| 21.1 |
| 21.2 |
| 22.1 |
| 22.7 |
| 24.7 |
| 25.3 |
| 27.9 |
| 29.3 |

**Table 8: Results on residual moisture of copanlisib lyophilisate 60 mg after 12 months**

| **Batch no.** | **Storage conditions** | **Residual moisture [%]** | |
|---|---|---|---|
| | | Specification max.2.0 % | |
| | | **Initial** | **12 months** |
| Batch A | 30 °C/75 % RH | 0.6 | 0.9 |
| | 2-8°C | | 0.5 |
| Batch B | 30 °C/75 % RH | 0.6 | 0.9 |
| | 25 °C/75 % RH | | 0.8 |
| | 2-8°C | | 0.6 |
| Batch F | 25 °C/60 % RH | 0.5 | 0.8 |

**Table 9: Results on residual moisture of Copanlisib lyophilisate 80 mg after 36 months**

| **Batch no.** | **Storage conditions** | **Residual moisture [%]** | |
|---|---|---|---|
| | | Specification max.2.0 % | |
| | | **Initial** | **36 months** |
| Batch G | 25 °C/60 % RH | 0.7 | 1.5 |
| | 2-8°C | | 1.0 |
| Batch D | 25 °C/75 % RH | 1.1 | 1.7 |
| | 2-8°C | | 1.1 |
| Batch E | 25 °C/60 % RH | 1.0 | n.d. |
| | 2-8°C | | 0.9 |

As no change in the amorphous state of the drug substance in the lyophilisate was detectable, the following humidity stress testing was conducted with copanlisib lyophilisate 60 mg for injection (Table 10). Cap and stopper of copanlisib lyophilisate vials were removed and the vials were stored at 25°C / 60 % RH up to 48 hours in order to increase the water content of the lyophilisate. The residual moisture was determined by Karl-Fischer titration. An equilibrium of 3.5% moisture is reached after 24 hours open storage.

Nevertheless, changes in the amorphous phase of the formulation were not detectable by XRPD up to 3.5% residual moisture and 48 hours open storage which demonstrates a very low crystallization tendency of the amorphous phase.

**Table 10 : Influence of residual moisture on solid state of copanlisib lyophilisate 60 mg for injection (Batch K)**

| **Open storage in climate chamber [h]** | **Residual water after open storage [%]** | **Solid State (XRPD)** |
|---|---|---|
| 0 | 0.4 | Mannitol Mod. III, drug substance amorphous |
| 0.5 | 0.8 | Mannitol Mod. III, drug substance amorphous |
| 1 | 1.4 | Mannitol Mod. III, drug substance amorphous |
| 1.5 | 1.6 | Mannitol Mod. III, drug substance amorphous |
| 2 | 1.7 | Mannitol Mod. III, drug substance amorphous |
| 3 | 2.4 | Mannitol Mod. III, drug substance amorphous |
| 4 | 3.0 | Mannitol Mod. III, drug substance amorphous |
| 24 | 3.6 | Mannitol Mod. III, drug substance amorphous |
| 48 | 3.5 | Mannitol Mod. III, drug substance amorphous |

### EXAMPLE 3 : Method of reconstituting a stable, lyophilized, copanlisib-containing solid, in particular powder or cake, suitable for dilution and for therapeutic applications.

Said method is hereinafter referred to as "reconstitution method" and the stable, reconstituted, copanlisib-containing solution of increased solubility, suitable for further dilution and for therapeutic applications, thus produced is hereinafter referred to as "reconstituted solution".

### Reconstituted (15mg/ml, copanlisib, free base) and diluted solution (0.56 mg/ml, copanlisib, free base)

Lyophilisates manufactured in the lab were reconstituted without removing the stopper with aqueous sodium chloride solution 0.9% resulting in a solution of 15mg/ml copanlisib, free base.

According to the results of the copanlisib bulk solution, the effect temperature was investigated also for the reconstituted solution (Table 11).

**Table 11 : Effect of temperature on physical stability of reconstituted solution (15mg/ml copanlisib, free base), batch H (in-use stability of reconstituted solution) :**

| **Observations** | **20°C** | **4°C** |
|---|---|---|
| | vial no. I | vial no. III |
| **Start** | clear solution | clear solution |
| **1 day** | clear solution | clear solution |
| **2 days** | clear solution | clear solution |
| **5 days** | clear solution | clear solution |
| **6 days** | clear solution | clear solution |
| **7 days** | clear solution | clear solution |

### EXAMPLE 4 : Copanlisib in Patients with Relapsed or Refractory Indolent B-cell Lymphoma.

**Methods:** Patients with indolent B-cell non-Hodgkin lymphoma (4 subtypes: follicular (FL), marginal zone (MZL), small lymphocytic (SLL) and lymphoplasmacytoid/Waldenström macroglobulinemia (LPL-WM)) and relapsed after, or refractory to, ≥2 prior lines of treatment were eligible. Previous treatment had to include rituximab and an alkylating agent. Copanlisib (60 mg, I.V.) was intermittently administered on days 1, 8 and 15 of a 28-day cycle. The primary efficacy endpoint was objective tumor response rate (ORR) as assessed per independent radiologic review (Cheson et al., JCO 20:579, 2007).

**Results:** The full analysis set comprised 142 patients, of which 141 patients had indolent lymphoma (FL/MZL/SLL/LPL-WM: 104/23/8/6). At the time of primary analysis, median duration of treatment was 22 weeks (range 1-105); 46 patients remained on treatment. The most common treatment-related adverse events (AEs) (all grade/grade 3+) were transient hyperglycemia (49%/40%) and hypertension (29%/23%). Other AEs of interest included neutropenia (25%/19%), diarrhea (18%/4%), lung infection (14%/11%), pneumonitis (7%/1.4%), and colitis (0.7%/0.7%). No colonic perforations occurred. There were two non-fatal opportunistic infections. Laboratory toxicities of interest were principally grade-1, including alanine aminotransferase (23% all-grade/19% grade-1) and aspartate aminotransferase (28%/25%). There were 6 deaths, 3 of which were attributed to copanlisib: lung infection, respiratory failure, and a thromboembolic event.

The overall response rate (ORR) was 59.2%, including 12.0% complete response (CR) and 47.2% partial response (PR), with stable disease in 29.6% of patients and progressive disease in 2.1% of patients. In the FL subset, the ORR was 58.7%, including 14.4% CR and 44.2% PR. In the MZL subset, the ORR was 69.6%, including 8.7% CR and 60.9% PR. The estimated Kaplan-Meier (KM) median duration of response in the full analysis set was 687 days (range 0-687) and 370 days (range 0-687) in the FL subset. The KM-estimate of median PFS was 340 days (range 0-736). Median overall survival had not yet been reached.

**Conclusions:** Treatment of patients with relapsed or refractory indolent B-cell lymphoma with copanlisib resulted in durable tumor responses. Administration of copanlisib had a manageable safety profile, with low rates of severe hepatic enzymopathy, diarrhea or inflammatory events, as well as low rates of opportunistic infections, fatal infections or other fatal SAEs.

In accordance with a seventh aspect, the present invention relates to a stable, reconstituted, copanlisib-containing solution of increased solubility, suitable for further dilution and for therapeutic applications, said reconstituted solution comprising :
- a therapeutic dose of copanlisib in one or two containers, particularly one sealed container,
- one or more pH-adjusting agents, particularly a buffering agent which in particular comprises a weak acid, such as citric acid for example, and a strong base, such as sodium hydroxide, for example, and
- a bulking agent,
said reconstituted solution having been prepared with a suitable diluent, such as sterile water for injection or sterile aqueous sodium chloride solution, particularly 0.9% aqueous sodium chloride solution, for example,
said reconstituted solution having a pH of between 4 and 5 (inclusive),
for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

In accordance with an embodiment of the seventh aspect of the present invention, said copanlisib-containing solution is defined in any one of the aspects described supra.

In accordance with an eighth aspect, the present invention relates to a stable, reconstituted, copanlisib-containing solution of increased solubility, suitable for further dilution and for therapeutic applications, said reconstituted solution comprising :
- a therapeutic dose of copanlisib in one or two containers, particularly one sealed container,
- one or more pH-adjusting agents, particularly a buffering agent which in particular comprises a weak acid, such as citric acid for example, and a strong base, such as sodium hydroxide, for example, and
- a bulking agent,
said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

In accordance with a ninth aspect, the present invention relates to a stable, reconstituted, copanlisib-containing solution of increased solubility, suitable for further dilution and for therapeutic applications, said reconstituted solution comprising :
- a therapeutic dose of copanlisib in one or two containers, particularly one sealed container,
- one or more pH-adjusting agents, particularly a buffering agent which in particular comprises a weak acid, such as citric acid for example, and a strong base, such as sodium hydroxide, for example, and
- a bulking agent,
said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
for the preparation of a medicament for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

In accordance with a tenth aspect, the present invention relates to a method of treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), in a subject, comprising administering to said subject a stable, reconstituted, copanlisib-containing solution of increased solubility, suitable for further dilution and for therapeutic applications, said reconstituted solution comprising :
- a therapeutic dose of copanlisib in one or two containers, particularly one sealed container,
- one or more pH-adjusting agents, particularly a buffering agent which in particular comprises a weak acid, such as citric acid for example, and a strong base, such as sodium hydroxide, for example, and
- a bulking agent,
said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
as monotherapy or in combination with one or more immunotherapeutic, chemotherapeutic or immuno-chemotherapeutic anti-cancer agents.

In accordance with an embodiment of the eighth, ninth or tenth aspect of the present invention, said copanlisib-containing solution is defined in any one of the aspects described supra.

In accordance with an embodiment of the eighth, ninth or tenth aspect of the present invention, said copanlisib-containing solution contains 30, 45, 60 or 80 mg of copanlisib.

In accordance with an embodiment of the eighth, ninth or tenth aspect of the present invention, said copanlisib-containing solution contains 60 mg of copanlisib.

In accordance with an embodiment of the eighth, ninth or tenth aspect of the present invention, said use of said copanlisib-containing solution is effected by administering a fixed dose of 60 mg copanlisib to a patient as a 1-hour intravenous infusion on days 1, 8, and 15 of a 28-day treatment cycle on an intermittent schedule (three weeks on and one week off).

In accordance with an embodiment of the eighth, ninth or tenth aspect of the present invention, said copanlisib-containing solution is administered in combination with a chemotherapeutic anti-cancer agent, particularly a chemotherapeutic anti-cancer agent selected from the group consisting of :
131I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (1231), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, rucaparib, samarium (153Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

The above-mentioned administration is accomplished orally or parenterally. For copanlisib, parenteral routes of administration are more preferred. Methods of parenteral delivery include topical, intra-arterial (directly to the tumor), intramuscular, subcutaneous, intramedullary, intrathecal, intraventricular, intravenous, intraperitoneal, or intranasal administration. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Ed. Maack Publishing Co, Easton, Pa.).

Formulations of the invention may be administered using an injector, a pump, a syringe, or any other devices/infusion devices known in the art as well as by gravity. A needle or a catheter may be used for introducing the formulations of the present invention into the body of a patient via certain parenteral routes.

Pharmaceutical compositions suitable for use in the present invention include compositions wherein the active ingredients are contained in an effective amount to achieve the intended purpose, i.e. treatment of a particular disease. The determination of an effective dose is well within the capability of those skilled in the art.

For copanlisib, the therapeutically effective dose can be estimated initially either in cell culture assays, e.g., neoplastic cells, or in animal models, usually mice, rabbits, dogs, pigs or monkeys. The animal model is also used to achieve a desirable concentration range and route of administration. Such information can then be used to determine useful doses and routes for administration in humans.

The copanlisib-containing compositions of this invention are formulated to be acceptable in a therapeutic application. "Therapeutic application" refers to treatments involving administration to a subject in need of treatment a therapeutically effective amount of copanlisib. A "therapeutically effective amount" hereby is defined as the amount of copanlisib that is of sufficient quantity to induce tumor cell death by apoptosis and inhibition of proliferation of primary malignant B cell line either as a single dose or according to a multiple dose regimen, alone or in combination with other agents, which leads to the alleviation of an adverse condition, yet which amount is toxicologically tolerable. The subject may be a human or non-human animal (e.g., rabbit, rat, mouse, dog, monkey or other lower-order primate).

The exact dosage can be chosen by the individual physician in view of the patient to be treated. Dosage and administration are adjusted to provide sufficient levels of the active moiety or to maintain the desired effect. Additional factors that may be taken into account include the severity of the disease state, e.g., tumor size and location; age, weight and gender of the patient; diet, time and frequency of administration, drug combination(s), reaction sensitivities, and tolerance/response to therapy.

Long acting pharmaceutical compositions might be administered for example every 3 to 4 days, every week, once every two weeks, or once every three weeks, depending on half-life and clearance rate of the particular formulation.

In accordance with the present invention, copanlisib-containing compositions can be administered as a fixed dose of 30, 45, 60 or 80 mg copanlisib to a patient as a 1-hour intravenous infusion on days 1, 8, and 15 of a 28-day treatment cycle on an intermittent schedule (three weeks on and one week off).

In accordance with the present invention, copanlisib-containing compositions are administered as a fixed dose of 60 mg copanlisib to a patient as a 1-hour intravenous infusion on days 1, 8, and 15 of a 28-day treatment cycle on an intermittent schedule (three weeks on and one week off).

In accordance with an eleventh aspect, the present invention relates to :
Item 1. A stable, reconstituted, copanlisib-containing solution of increased solubility, suitable for further dilution and for therapeutic applications, said reconstituted solution comprising :
   - copanlisib, particularly in an amount suitable as a therapeutic dose, in one or two containers, particularly one sealed container,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having been prepared with a suitable diluent, such as sterile water for injection or sterile aqueous sodium chloride solution, particularly 0.9% aqueous sodium chloride solution, for example,
   said reconstituted solution having a pH of between 4 and 5 (inclusive).
Item 2. The reconstituted copanlisib-containing solution according to item 1, wherein said therapeutic dose is 30, 45, 60 or 80 mg of copanlisib.
Item 3. The reconstituted copanlisib-containing solution according to item 1 or 2, wherein said therapeutic dose is 60 mg of copanlisib.
Item 4. The reconstituted copanlisib-containing solution according to any one of items 1 to 3, wherein said pH-adjusting agent is a buffering agent.
Item 5. The reconstituted copanlisib-containing solution according to any one of items 1 to 4, wherein said buffering agent is citric acid and sodium hydroxide.
Item 6. The reconstituted copanlisib-containing solution according to any one of items 1 to 5, which contains citric acid in an amount of 0.08 - 15.0% w/v, particularly 0.15-3.0% w/v, in particular 0.15% w/v, of said reconstituted solution.
Item 7. The reconstituted copanlisib-containing solution according to any one of items 1 to 6, which contains sodium hydroxide in an amount of 0-0.3% w/v, particularly 0.14-0.18 % w/v, in particular 0.16 % w/v, of said reconstituted solution.
Item 8. The reconstituted copanlisib-containing solution according to any one of items 1 to 7, which contains sodium hydroxide in an amount of 0.01-0.3% w/v, particularly 0.14-0.18 % w/v, in particular 0.16 % w/v, of said reconstituted solution.
Item 9. The reconstituted copanlisib-containing solution according to any one of items 1 to 8, wherein said bulking agent is mannitol.
Item 10. The reconstituted copanlisib-containing solution according to item 9, which contains mannitol in an amount of 1.5-24.0% w/v, particularly 1.5-16.0% w/v, in particular 3.0% w/v, of said reconstituted solution.
Item 11. The reconstituted copanlisib-containing solution according to any one of items 1 to 10, wherein said diluent is sterile aqueous sodium chloride solution, particularly 0.9% aqueous sodium chloride solution.
Item 12. The reconstituted copanlisib-containing solution according to any one of items 1 to 11, which contains said copanlisib free base in an amount of 0.5-16.0% w/v, particularly 1.0-8.0% w/v, in particular 1.5% w/v, of said reconstituted solution.
Item 13. The reconstituted copanlisib-containing solution according to any one of items 9 to 12, wherein :
   - said therapeutic dose of copanlisib is in an amount of 1.5% w/v of said reconstituted solution,
   - said citric acid is in an amount of 0.15% w/v of said reconstituted solution,
   - said sodium hydroxide is in an amount of 0.16 % w/v, of said reconstituted solution, and
   - said mannitol is in an amount of 3.0% w/v, of said reconstituted solution,
   said diluent being 0.9% aqueous sodium chloride solution,
   said copanlisib-containing solution having a pH of between 4 and 5 (inclusive).
Item 14. The reconstituted copanlisib-containing solution according to any one of items 9 to 13, wherein :
   - said therapeutic dose of copanlisib is in an amount of 68.4 mg,
   - said citric acid is in an amount of 6.6 mg,
   - said sodium hydroxide is in an amount of 7.2 mg, and
   - said mannitol is in an amount of 136.8 mg,
   - said diluent, which is 0.9% aqueous sodium chloride solution, is in an amount of 4.4ml,
   said reconstituted copanlisib-containing solution having a pH of between 4 and 5 (inclusive),
   said reconstituted copanlisib-containing solution having a volume of 4.55 ml.
Item 15. The reconstituted copanlisib-containing solution according to any one of items 1 to 14, which is contained in one container, particularly a sealed container, particularly an injection vial, more particularly a 6 ml injection vial.
Item 16. The reconstituted copanlisib-containing solution according to any one of items 1 to 15, containing copanlisib in an amount of 15 mg/ml.
Item 17. The reconstituted copanlisib-containing solution according to any one of items 1 to 16, which contains 60 mg of copanlisib, particularly in a volume of 4 ml.
Item 18. The reconstituted copanlisib-containing solution according to any one of items 1 to 17, which is further diluted with a suitable diluent, such as sterile water for injection or sterile aqueous sodium chloride solution, particularly 0.9% aqueous sodium chloride solution, for example.
Item 19. The reconstituted copanlisib-containing solution according to item 18, wherein said suitable diluent is 0.9% aqueous sodium chloride solution, particularly in a volume of 100 ml. Item 20. The reconstituted copanlisib-containing solution according to item 19, which is contained in an infusion bag, particularly made of polyethylene (PE), polypropylene (PP), polyvinylchloride (PVC), or ethylene vinyl acetate (EVA).
Item 21. A method of reconstituting a stable, lyophilized, copanlisib-containing solid, in particular powder or cake, suitable for dilution and for therapeutic applications, said stable, lyophilized solid, in particular powder, comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example,
   said reconstitution method comprising :
   - adding, to said stable, lyophilized solid, in particular powder, in a suitable container, particularly a sealed container, particularly an injection vial, more particularly a 6 ml injection vial, a diluent, such as sterile water for injection or sterile aqueous sodium chloride solution, particularly 0.9% aqueous sodium chloride solution, for example ;
   said method thus providing a stable, reconstituted, copanlisib-containing solution, suitable for further dilution and for therapeutic applications, said reconstituted solution comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having a pH of between 4 and 5 (inclusive).
Item 22. The reconstitution method according to item 21, wherein said copanlisib-containing solution produced therefrom is defined in any one of items 1 to 21.
Item 23. A stable, lyophilized, copanlisib-containing solid, in particular powder or cake, suitable for dilution and for therapeutic applications, said solid, in particular powder or cake, comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example.
Item 24. The lyophilized, copanlisib-containing solid, in particular powder or cake, according to item 23, wherein said therapeutic dose is 30, 45, 60 or 80 mg of copanlisib.
Item 25. The lyophilized, copanlisib-containing solid, in particular powder or cake, according to item 23, wherein said therapeutic dose is 60 mg of copanlisib.
Item 26. The lyophilized, copanlisib-containing solid, in particular powder or cake, according to item 23 or 24, wherein said pH-adjusting agent is a buffering agent.
Item 27. The lyophilized, copanlisib-containing solid, in particular powder or cake, according to any one of items 23 to 26, wherein said buffering agent is citric acid and sodium hydroxide.
Item 28. The lyophilized, copanlisib-containing solid, in particular powder or cake, according to any one of items 23 to 27, which contains citric acid in an amount of 0.7- 75 % w/w, particularly 1.5-30% w/w, in particular 2.98% w/w, of said lyophilized, copanlisib-containing solid.
Item 29. The lyophilized, copanlisib-containing solid, in particular powder or cake, according to any one of items 23 to 28, which contains sodium hydroxide in an amount of 0-6% w/w, particularly 3.3-4.0 % w/w, in particular 3.7% w/w, of said lyophilized, copanlisib-containing solid.
Item 30. The lyophilized, copanlisib-containing solid, in particular powder or cake, according to any one of items 23 to 28, which contains sodium hydroxide in an amount of 0.01-6% w/w, particularly 3.3-4.0 % w/w, in particular 3.7% w/w, of said lyophilized, copanlisib-containing solid.
Item 31. The lyophilized, copanlisib-containing solid, in particular powder or cake, according to any one of items 23 to 30, wherein said bulking agent is mannitol.
Item 32. The lyophilized, copanlisib-containing solid, in particular powder or cake, according to item 31, which contains mannitol in an amount of 15.4-82.1% w/w, particularly 30.8-82.1% w/w, in particular 61.6% w/w, of said lyophilized, copanlisib-containing solid.
Item 33. The lyophilized, copanlisib-containing solid, in particular powder or cake, according to any one of items 23 to 32, which contains said copanlisib free base in an amount of 7.7-92.4% w/w, particularly 7.7-61.6% w/w, in particular 30.8% w/w, of lyophilized, copanlisib-containing solid.
Item 34. The lyophilized, copanlisib-containing solid, in particular powder or cake, according to any one of items 23 to 33, wherein :
   - said therapeutic dose of copanlisib is in an amount of 30.8% w/w of said lyophilized, copanlisib-containing solid,
   - said citric acid is in an amount of 2.98% w/w of said lyophilized, copanlisib-containing solid,
   - said sodium hydroxide is in an amount of 3.7% w/w of said lyophilized, copanlisib-containing solid, and
   - said mannitol is in an amount of 61.6% w/w, of said lyophilized, copanlisib-containing solid,
   said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).
Item 35. The lyophilized, copanlisib-containing solid, in particular powder or cake, according to any one of items 23 to 34, wherein :
   - said therapeutic dose of copanlisib is in an amount of 30, 45 or 60 mg,
   - said citric acid is in an amount of 5.8mg,
   - said sodium hydroxide is in an amount of 6.3mg, and
   - said mannitol is in an amount of 120mg,
   said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).
Item 36. The lyophilized, copanlisib-containing solid, in particular powder or cake, according to any one of items 23 to 34, wherein :
   - said therapeutic dose of copanlisib is in an amount of 80 mg,
   - said citric acid is in an amount of 7.7 mg,
   - said sodium hydroxide is in an amount of 8.4 mg, and
   - said mannitol is in an amount of 160 mg,
   said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).
Item 37. The lyophilized, copanlisib-containing solid, in particular powder or cake, according to any one of items 23 to 36, which is contained in one container, particularly a sealed container, particularly an injection vial, more particularly a 6 ml injection vial.
Item 38. The lyophilized, copanlisib-containing solid, in particular powder or cake, according to any one of items 23 to 37, containing copanlisib in an amount of 68.4 mg.
Item 39. A method of lyophilizing a stable, copanlisib-containing aqueous bulk solution suitable for lyophilisation and for therapeutic applications, said composition comprising:
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said bulk solution having a pH of between 4 and 5 (inclusive),
   said lyophilisation method including : freezing, annealing, primary and secondary drying steps;
   said method thus providing a stable, lyophilized, copanlisib-containing solid, in particular powder or cake, suitable for dilution and for therapeutic applications, said solid, in particular powder or cake, comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example.
Item 40. A stable, copanlisib-containing aqueous bulk solution of increased solubility which is directly suitable for lyophilisation, said bulk solution comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said bulk solution having a pH of between 4 and 5 (inclusive).
Item 41. The bulk copanlisib-containing solution according to item 40, wherein said therapeutic dose is 30, 45, 60 or 80 mg of copanlisib.
Item 42. The bulk copanlisib-containing solution according to item 40, wherein said therapeutic dose is 60 mg of copanlisib.
Item 43. The bulk copanlisib-containing solution according to any one of items 40 to 42, wherein said pH-adjusting agent is a buffering agent.
Item 44. The bulk copanlisib-containing solution according to any one of items 40 to 43, wherein said buffering agent is citric acid and sodium hydroxide.
Item 45. The bulk copanlisib-containing solution according to any one of items 40 to 44, which contains citric acid in an amount of 0.18 - 18.5% w/w, particularly 0.18-3.7% w/w, in particular 0.37% w/w, of said bulk solution.
Item 46. The bulk copanlisib-containing solution according to any one of items 40 to 45, which contains sodium hydroxide in an amount of 0-0.8% w/w, particularly 0.3-0.5% w/w, in particular 0.4 % w/w, of said bulk solution.
Item 47. The bulk copanlisib-containing solution according to any one of items 40 to 46, wherein said bulking agent is mannitol.
Item 48. The bulk copanlisib-containing solution according to item 47, which contains mannitol in an amount of 3.0-23.1% w/w, particularly 3.0-15.4% w/w, in particular 7.7% w/w, of said bulk solution.
Item 49. The bulk copanlisib-containing solution according to any one of items 40 to 48, which contains said copanlisib free base in an amount of 0.5-15.2% w/w, particularly 1.0-7.6% w/w, in particular 3.8% w/w, of said bulk solution.
Item 50. The bulk copanlisib-containing solution according to any one of items 40 to 49, wherein :
   - said therapeutic dose of copanlisib is in an amount of 3.8% w/w of said bulk solution,
   - said citric acid is in an amount of 0.37% w/w of said bulk solution,
   - said sodium hydroxide is in an amount of 0.4 % w/w, of said bulk solution, and
   - said mannitol is in an amount of 7.7% w/w, of said bulk solution,
   said copanlisib-containing solution having a pH of between 4 and 5 (inclusive).
Item 51. The bulk copanlisib-containing solution according to any one of items 40 to 50, wherein :
   - said therapeutic dose of copanlisib is in an amount of 68.4 mg,
   - said citric acid is in an amount of 6.6 mg,
   - said sodium hydroxide is in an amount of 7.2 mg, and
   - said mannitol is in an amount of 136.8 mg,
   said bulk copanlisib-containing solution having a pH of between 4 and 5 (inclusive),
   said bulk copanlisib-containing solution having a volume of 1.71 ml.
Item 52. The bulk copanlisib-containing solution according to any one of items 40 to 51, which contains copanlisib in an amount of 40 mg/ml.
Item 53. The bulk copanlisib-containing solution according to any one of items 40 to 52, which contains 68.4 mg of copanlisib in a volume of 1.71 ml.
Item 54. The bulk copanlisib-containing solution according to any one of items 40 to 53, which contains 60 mg of copanlisib in a volume of 1.5 ml.
Item 55. The bulk copanlisib-containing solution according to any one of items 40 to 54, which is further diluted with a suitable diluent, such as sterile water for injection or sterile aqueous sodium chloride solution, particularly 0.9% aqueous sodium chloride solution, for example.
Item 56. The bulk copanlisib-containing solution according to item 55, wherein :
   - said therapeutic dose of copanlisib is in an amount of 68.4 mg,
   - said citric acid is in an amount of 6.6 mg,
   - said sodium hydroxide is in an amount of 7.2 mg, and
   - said mannitol is in an amount of 136.8 mg,
   is further diluted with :
   - a diluent, which is 0.9% aqueous sodium chloride solution, in an amount of 4.4 ml,
   said bulk copanlisib-containing solution having a pH of between 4 and 5 (inclusive),
   said bulk copanlisib-containing solution having a volume of 1.71 ml.
Item 57. The bulk copanlisib-containing solution according to item 55 or 56, which is further diluted with a suitable diluent, wherein said suitable diluent is 0.9% aqueous sodium chloride solution, particularly in a volume of 100 ml.
Item 58. The bulk copanlisib-containing solution according to any one of items 55 to 57, which is contained in an infusion bag, particularly made of polyethylene (PE), polypropylene (PP), polyvinylchloride (PVC), or ethylene vinyl acetate (EVA).
Item 59. A method of preparing a stable, copanlisib-containing aqueous bulk solution suitable for lyophilisation and for therapeutic applications, said bulk solution comprising :
   - copanlisib, particularly in an amount suitable as a therapeutic dose,
   - one or more pH-adjusting agents, particularly a buffering agent which in particular comprises a weak acid, such as citric acid for example, and a strong base, such as sodium hydroxide, for example, and
   - a bulking agent,
   said bulk solution having a pH of between 4 and 5 (inclusive),
   said method comprising :
   - charging a suitable vessel with water for injection ;
   - transferring copanlisib, in the form of a free base, or a salt, such as a hydrochloride, in particular a monohydrochloride or a dihydrochloride, or a tosylate, for example, to said vessel;
   - allowing said copanlisib to dissolve, optionally by stirring for example, at a pH of between 2 and 3 (inclusive), thus forming a copanlisib-containing solution ;
   - adding said buffering agent and said bulking agent to said copanlisib-containing solution;
   - allowing said buffering agent and said bulking agent to dissolve, optionally by stirring for example;
   - adjusting the pH of the thus-formed solution to a pH of 4 to 5 with an appropriate amount of sodium hydroxide solution, thus forming a bulk solution ;
   - filtering said bulk solution in a sterile manner,
   thus providing a stable, copanlisib-containing aqueous bulk solution suitable for direct lyophilisation and therapeutic applications ; and
   - filling the thus-formed filtered bulk solution in one or more vials.
Item 60. The method according to item 59, wherein said copanlisib transferred to said vessel is copanlisib dihydrochloride.
Item 61. A stable, reconstituted, copanlisib-containing solution of increased solubility, suitable for further dilution and for therapeutic applications, said reconstituted solution comprising :
   - a therapeutic dose of copanlisib in one or two containers, particularly one sealed container,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having been prepared with a suitable diluent, such as sterile water for injection or sterile aqueous sodium chloride solution, particularly 0.9% aqueous sodium chloride solution, for example,
   said reconstituted solution having a pH of between 4 and 5 (inclusive),
   for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL),
   as monotherapy or in combination with one or more immunotherapeutic, chemotherapeutic or immuno-chemotherapeutic anti-cancer agents.
Item 62. Use according to item 61, wherein said copanlisib-containing solution is defined in any one of items 1 to 20.
Item 63. Use of a stable, reconstituted, copanlisib-containing solution of increased solubility, suitable for further dilution and for therapeutic applications, said reconstituted solution comprising :
   - a therapeutic dose of copanlisib in one or two containers, particularly one sealed container,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
   for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL),
   as monotherapy or in combination with one or more immunotherapeutic, chemotherapeutic or immuno-chemotherapeutic anti-cancer agents.
Item 64. Use of a stable, reconstituted, copanlisib-containing solution of increased solubility, suitable for further dilution and for therapeutic applications, said reconstituted solution comprising :
   - a therapeutic dose of copanlisib in one or two containers, particularly one sealed container,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
   for the preparation of a medicament for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.
Item 65. A method of treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL),
   in a subject, comprising administering to said subject a stable, reconstituted, copanlisib-containing solution of increased solubility, suitable for further dilution and for therapeutic applications, said reconstituted solution comprising :
   - a therapeutic dose of copanlisib in one or two containers, particularly one sealed container,
   - one or more pH-adjusting agents, particularly a buffering agent which in particular comprises a weak acid, such as citric acid for example, and a strong base, such as sodium hydroxide, for example, and
   - a bulking agent,
   said reconstituted solution having been prepared with a suitable diluent, such as sterile water for injection or sterile aqueous sodium chloride solution, particularly 0.9% aqueous sodium chloride solution, for example, said reconstituted solution having a pH of between 4 and 5 (inclusive),
   as monotherapy or in combination with one or more immunotherapeutic, chemotherapeutic or immuno-chemotherapeutic anti-cancer agents.
Item 66. Use according to item 63 or 64, or the method according to item 65, wherein said copanlisib-containing solution is defined in any one of items 1 to 20.
Item 67. Use according to any one of items 63 or 64, or the method according to item 65, wherein said copanlisib-containing solution contains 60 mg of copanlisib.
Item 68. Use according to item 67, wherein said copanlisib-containing solution is of a volume of 4.0 ml.
Item 69. Use according to item 68, said use is effected by administering a fixed dose of 60 mg copanlisib to a patient as a 1-hour intravenous infusion on days 1, 8, and 15 of a 28-day treatment cycle on an intermittent schedule (three weeks on and one week off).
Item 70. Use according to items 63 or 64, or the method according to item 65, wherein said solution is administered in combination with a chemotherapeutic anti-cancer agent.
Item 71. Use according to item 70, wherein said chemotherapeutic anti-cancer agent is selected from the group consisting of :
   131I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (1231), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, rucaparib, samarium (153Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.,

## Claims

1. A stable, lyophilized, copanlisib-containing solid, in particular powder or cake, suitable for dilution and for therapeutic applications, said solid, in particular powder or cake, comprising :
• copanlisib, particularly in an amount suitable as a therapeutic dose,
• one or more pH-adjusting agents, particularly a buffering agent which in particular comprises a weak acid, such as citric acid for example, and a strong base, such as sodium hydroxide, for example, and
• a bulking agent,
said solid, in particular powder or cake, having a pH of between 4 and 5 (inclusive) when reconstituted with a suitable diluent, such as sterile water for injection or sterile aqueous sodium chloride solution, particularly 0.9% aqueous sodium chloride solution, for example.

2. The lyophilized, copanlisib-containing solid, in particular powder or cake, according to claim 1, wherein said therapeutic dose is 30, 45 or 60 mg of copanlisib.

3. The lyophilized, copanlisib-containing solid, in particular powder or cake, according to claim 1 or 2, wherein said pH-adjusting agent is a buffering agent.

4. The lyophilized, copanlisib-containing solid, in particular powder or cake, according to any one of claims 1 to 3, wherein said buffering agent is citric acid and sodium hydroxide.

5. The lyophilized, copanlisib-containing solid, in particular powder or cake, according to any one of claims 1 to 4, which contains citric acid in an amount of 0.7- 75 % w/w, particularly 1.5-30% w/w, in particular 2.98% w/w, of said lyophilized, copanlisib-containing solid.

6. The lyophilized, copanlisib-containing solid, in particular powder or cake, according to any one of claims 1 to 5, which contains sodium hydroxide in an amount of 0-6% w/w, particularly 3.3-4.0 % w/w, in particular 3.7% w/w, of said lyophilized, copanlisib-containing solid.

7. The lyophilized, copanlisib-containing solid, in particular powder or cake, according to any one of claims 1 to 6, which contains sodium hydroxide in an amount of 0.01-6% w/w, particularly 3.3-4.0 % w/w, in particular 3.7% w/w, of said lyophilized, copanlisib-containing solid.

8. The lyophilized, copanlisib-containing solid, in particular powder or cake, according to any one of claims 1 to 7, wherein said bulking agent is mannitol.

9. The lyophilized, copanlisib-containing solid, in particular powder or cake, according to claim 8, which contains mannitol in an amount of 15.4-82.1% w/w, particularly 30.8-82.1% w/w, in particular 61.6% w/w, of said lyophilized, copanlisib-containing solid.

10. The lyophilized, copanlisib-containing solid, in particular powder or cake, according to any one of claims 1 to 9, which contains said copanlisib free base in an amount of 7.7-92.4% w/w, particularly 7.7-61.6% w/w, in particular 30.8% w/w, of lyophilized, copanlisib-containing solid.

11. The lyophilized, copanlisib-containing solid, in particular powder or cake, according to any one of claims 1 to 10, wherein :
• said therapeutic dose of copanlisib is in an amount of 30.8% w/w of said lyophilized, copanlisib-containing solid,
• said citric acid is in an amount of 2.98% w/w of said lyophilized, copanlisib-containing solid,
• said sodium hydroxide is in an amount of 3.7% w/w of said lyophilized, copanlisib-containing solid, and
• said mannitol is in an amount of 61.6% w/w, of said lyophilized, copanlisib-containing solid,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

12. The lyophilized, copanlisib-containing solid, in particular powder or cake, according to any one of claims 1 to 11, wherein :
• said therapeutic dose of copanlisib is in an amount of 30, 45 or 60 mg,
• said citric acid is in an amount of 5.8mg,
• said sodium hydroxide is in an amount of 6.3mg, and
• said mannitol is in an amount of 120mg,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

13. The lyophilized, copanlisib-containing solid, in particular powder or cake, according to any one of claims 1 to 11, wherein :
• said therapeutic dose of copanlisib is in an amount of 80 mg,
• said citric acid is in an amount of 7.7 mg,
• said sodium hydroxide is in an amount of 8.4 mg, and
• said mannitol is in an amount of 160 mg,
said lyophilized, copanlisib-containing solid, in particular powder or cake, when reconstituted with a suitable diluent, such as sterile water for injection or sterile aqueous sodium chloride solution, particularly 0.9% aqueous sodium chloride solution, for example, having a pH of between 4 and 5 (inclusive).

14. The lyophilized, copanlisib-containing solid, in particular powder or cake, according to any one of claims 1 to 13, which is contained in one container, particularly a sealed container, particularly an injection vial, more particularly a 6 ml injection vial.

15. The lyophilized, copanlisib-containing solid, in particular powder or cake, according to any one of claims 1 to 14, containing copanlisib in an amount of 68.4 mg.

16. A method of preparing the lyophilized, copanlisib-containing solid, in particular powder or cake, according to any one of claims 1 to 15,
said method including :
• preparing a stable, copanlisib-containing aqueous bulk solution of increased solubility which is directly suitable for lyophilisation, said bulk solution comprising :
∘ copanlisib, particularly in an amount suitable as a therapeutic dose,
∘ one or more pH-adjusting agents, particularly a buffering agent which in particular comprises a weak acid, such as citric acid for example, and a strong base, such as sodium hydroxide, for example, and
∘ a bulking agent,
said bulk solution having a pH of between 4 and 5 (inclusive).
• freezing, annealing, primary and secondary drying steps ;
said method thus providing a stable, lyophilized, copanlisib-containing solid, in particular powder or cake, suitable for dilution and for therapeutic applications, according to any one of claims 1 to 15.

17. A stable, copanlisib-containing aqueous bulk solution of increased solubility which is directly suitable for lyophilisation, said bulk solution comprising :
• copanlisib, particularly in an amount suitable as a therapeutic dose,
• one or more pH-adjusting agents, particularly a buffering agent which in particular comprises a weak acid, such as citric acid for example, and a strong base, such as sodium hydroxide, for example, and
• a bulking agent,
said bulk solution having a pH of between 4 and 5 (inclusive),
which particularly contains copanlisib in an amount of 40 mg/ml.

18. The bulk copanlisib-containing solution according to claim 17, wherein :
• said therapeutic dose of copanlisib is in an amount of 3.8% w/w of said bulk solution,
• said citric acid is in an amount of 0.37% w/w of said bulk solution,
• said sodium hydroxide is in an amount of 0.4 % w/w, of said bulk solution, and
• said bulking agent, which is mannitol is in an amount of 7.7% w/w, of said bulk solution,
said copanlisib-containing solution having a pH of between 4 and 5 (inclusive).

19. A method of preparing the stable, copanlisib-containing aqueous bulk solution according to claim 17 or 18,
said method comprising :
• charging a suitable vessel with water for injection ;
• transferring copanlisib, in the form of a free base, or a salt, such as a hydrochloride, in particular a monohydrochloride or a dihydrochloride, or a tosylate, for example, to said vessel;
• allowing said copanlisib to dissolve, optionally by stirring for example, at a pH of between 2 and 3 (inclusive), thus forming a copanlisib-containing solution ;
• adding said buffering agent and said bulking agent to said copanlisib-containing solution;
• allowing said buffering agent and said bulking agent to dissolve, optionally by stirring for example;
• adjusting the pH of the thus-formed solution to a pH of 4 to 5 with an appropriate amount of sodium hydroxide solution, thus forming a bulk solution ;
• filtering said bulk solution in a sterile manner,
thus providing a stable, copanlisib-containing aqueous bulk solution suitable for direct lyophilisation and therapeutic applications ; and
• filling the thus-formed filtered bulk solution in one or more vials.

20. The method according to claim 19, wherein said copanlisib transferred to said vessel is copanlisib dihydrochloride.

21. The lyophilized, copanlisib-containing solid, in particular powder or cake, according to any one of claims 1 to 15, which is prepared according to the method according to claim 16, particularly from a stable, copanlisib-containing aqueous bulk solution according to claim 17 or 18, said bulk solution having been prepared in particular by the method according to claim 19 or 20.

22. A stable, reconstituted, copanlisib-containing solution of increased solubility, suitable for further dilution and for therapeutic applications, said reconstituted solution comprising :
• a lyophilized, copanlisib-containing solid, in particular powder or cake, according to any one of claims 1 to 15 or 21, and
• a suitable diluent, such as sterile water for injection or sterile aqueous sodium chloride solution, particularly 0.9% aqueous sodium chloride solution, for example,
said reconstituted solution having a pH of between 4 and 5 (inclusive).

23. The reconstituted copanlisib-containing solution according to claim 22, which contains citric acid in an amount of 0.08 - 15.0% w/v, particularly 0.15-3.0% w/v, in particular 0.15% w/v, of said reconstituted solution.

24. The reconstituted copanlisib-containing solution according to claim 22 or 23, which contains sodium hydroxide in an amount of 0-0.3% w/v, particularly 0.14-0.18 % w/v, in particular 0.16 % w/v, of said reconstituted solution.

25. The reconstituted copanlisib-containing solution according to claim 22 or 23, which contains contains sodium hydroxide in an amount of 0.01-0.3% w/v, particularly 0.14-0.18 % w/v, in particular 0.16 % w/v, of said reconstituted solution.

26. The reconstituted copanlisib-containing solution according to any one of claims 22 to 25, which contains mannitol in an amount of 1.5-24.0% w/v, particularly 1.5-16.0% w/v, in particular 3.0% w/v, of said reconstituted solution.

27. The reconstituted copanlisib-containing solution according to any one of claims 22 to 25, which contains said copanlisib free base in an amount of 0.5-16.0% w/v, particularly 1.0-8.0% w/v, in particular 1.5% w/v, of said reconstituted solution.

28. The reconstituted copanlisib-containing solution according to any one of claims 22 to 27, wherein :
• said therapeutic dose of copanlisib is in an amount of 1.5% w/v of said reconstituted solution,
• said citric acid is in an amount of 0.15% w/v of said reconstituted solution,
• said sodium hydroxide is in an amount of 0.16 % w/v, of said reconstituted solution, and
• said mannitol is in an amount of 3.0% w/v, of said reconstituted solution,
said diluent being 0.9% aqueous sodium chloride solution,
said copanlisib-containing solution having a pH of between 4 and 5 (inclusive).

29. The reconstituted copanlisib-containing solution according to any one of claims 22 to 27, wherein :
• said therapeutic dose of copanlisib is in an amount of 68.4 mg,
• said citric acid is in an amount of 6.6 mg,
• said sodium hydroxide is in an amount of 7.2 mg, and
• said mannitol is in an amount of 136.8 mg,
• said diluent, which is 0.9% aqueous sodium chloride solution, is in an amount of 4.4ml,
said reconstituted copanlisib-containing solution having a pH of between 4 and 5 (inclusive),
said reconstituted copanlisib-containing solution having a volume of 4.55 ml.

30. The reconstituted copanlisib-containing solution according to any one of claims 22 to 28, which is contained in one container, particularly a sealed container, particularly an injection vial, more particularly a 6 ml injection vial.

31. The reconstituted copanlisib-containing solution according to any one of claims 22 to 30, containing copanlisib in an amount of 15 mg/ml.

32. The reconstituted copanlisib-containing solution according to any one of claims 22 to 31, which contains 60 mg of copanlisib, particularly in a volume of 4 ml.

33. The reconstituted copanlisib-containing solution according to any one of claims 22 to 32, which is further diluted with a suitable diluent, such as sterile water for injection or sterile aqueous sodium chloride solution, particularly 0.9% aqueous sodium chloride solution, for example.

34. The reconstituted copanlisib-containing solution according to claim 33, wherein said suitable diluent is 0.9% aqueous sodium chloride solution, particularly in a volume of 100 ml.

35. The reconstituted copanlisib-containing solution according to claim 34, which is contained in an infusion bag, particularly made of polyethylene (PE), polypropylene (PP), polyvinylchloride (PVC), or ethylene vinyl acetate (EVA).

36. A method of reconstituting the stable, lyophilized, copanlisib-containing solid, in particular powder or cake, suitable for dilution and for therapeutic applications, according to any one of claims 1 to 15 or 21, or as prepared by the method according to claim 16,
said reconstitution method comprising :
• adding, to said stable, lyophilized solid, in particular powder, in a suitable container, particularly a sealed container, particularly an injection vial, more particularly a 6 ml injection vial, a diluent, such as sterile water for injection or sterile aqueous sodium chloride solution, particularly 0.9% aqueous sodium chloride solution, for example ;
said method thus providing a stable, reconstituted, copanlisib-containing solution, suitable for further dilution and for therapeutic applications, according to any one of claims 22 to 35.

37. A lyophilized, copanlisib-containing solid, in particular powder or cake, according to any one of claims 1 to 15 or 21, or as prepared according to the method of claim 16, or a stable, reconstituted, copanlisib-containing solution of increased solubility, suitable for further dilution and for therapeutic applications, according to any one of claims 22 to 35, or as prepared according to the method of claim 36,
for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

38. Use of a lyophilized, copanlisib-containing solid, in particular powder or cake, according to any one of claims 1 to 15 or 21, or as prepared according to the method of claim 16, or a stable, reconstituted, copanlisib-containing solution of increased solubility, suitable for further dilution and for therapeutic applications, according to any one of claims 22 to 35, or as prepared according to the method of claim 36,
for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

39. Use of a lyophilized, copanlisib-containing solid, in particular powder or cake, according to any one of claims 1 to 15 or 21, or as prepared according to the method of claim 16, or a stable, reconstituted, copanlisib-containing solution of increased solubility, suitable for further dilution and for therapeutic applications, according to any one of claims 22 to 35, or as prepared according to the method of claim 36,
for the preparation of a medicament for the treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL), as monotherapy or in combination with one or more immunotherapeutic, chemotherapeutic or immuno-chemotherapeutic anti-cancer agents.

40. A method of treatment or prophylaxis of a cancer, particularly a non-Hodgkin's lymphoma (NHL), such as 1st line, 2nd line, or up to 9 prior treatments, relapsed, refractory, indolent NHL, in particular follicular lymphoma (FL), marginal zone lymphoma (MZL), small lymphocytic lymphoma (SLL), Lymphoplasmacytoid/Waldenström macroglobulinemia (LPL-WM), or aggressive NHL, in particular diffuse large B cell lymphoma (DLBCL), mantle cell lymphoma (MCL), peripheral T-cell lymphoma (PTCL), transformed lymphoma (TL), or chronic lymphocytic leukemia (CLL),
in a subject, comprising administering to said subject a stable, reconstituted, copanlisib-containing solution of increased solubility, suitable for further dilution and for therapeutic applications, and 5 (inclusive),
a stable, reconstituted, copanlisib-containing solution of increased solubility, suitable for further dilution and for therapeutic applications, according to any one of claims 22 to 35, or as prepared according to the method of claim 36,
as monotherapy or in combination with one or more immunotherapeutic, chemotherapeutic or immuno-chemotherapeutic anti-cancer agents.

41. Use according to claim 38 or 39, or the method according to claim 40, wherein said copanlisib-containing solution contains 60 mg of copanlisib.

42. Use according to claim 41, wherein said copanlisib-containing solution is of a volume of 4.0 ml.

43. Use according to claim 42, said use is effected by administering a fixed dose of 60 mg copanlisib to a patient as a 1-hour intravenous infusion on days 1, 8, and 15 of a 28-day treatment cycle on an intermittent schedule (three weeks on and one week off).

44. Use according to claims 38 or 39, or the method according to claim 40, or any one of claims 41 to 42, wherein said solution is administered in combination with a chemotherapeutic anti-cancer agent.

45. Use according to claim 44, wherein said chemotherapeutic anti-cancer agent is selected from the group consisting of :
131I-chTNT, abarelix, abiraterone, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, aprepitant, arcitumomab, arglabin, arsenic trioxide, asparaginase, atezolizumab, axitinib, azacitidine, basiliximab, belotecan, bendamustine, besilesomab, belinostat, bevacizumab, bexarotene, bicalutamide, bisantrene, bleomycin, blinatumomab, bortezomib, buserelin, bosutinib, brentuximab vedotin, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, eculizumab, edrecolomab, elliptinium acetate, elotuzumab, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (1231), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, necitumumab, nedaplatin, nelarabine, neridronic acid, netupitant/palonosetron, nivolumab, pentetreotide, nilotinib, nilutamide, nimorazole, nimotuzumab, nimustine, nintedanib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, risedronic acid, rhenium-186 etidronate, rituximab, rolapitant, romidepsin, romiplostim, romurtide, rucaparib, samarium (153Sm) lexidronam, sargramostim, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.
